# EUROPEAN PATENT APPLICATION

(11) **EP 4 083 200 A2**
(43) Date of publication of application: **02.11.2022**
(21) Application number: 22162860.5
(22) Date of filing: 18.03.2022
(51) Int. Cl.: C12N 11/04, B01D 53/85, C12M 1/107, C12M 1/12, C12P 3/00, C12P 7/02, B01D 53/72

(54) **SYSTEMS AND METHODS FOR REMOVING METHANE FOR A GAS STREAM**

(30) Priority: 05.04.2021 US 202163170860 P; 20.05.2021 US 202163190922 P; 20.12.2021 US 202117555684
(71) Applicant: Palo Alto Research Center Incorporated, Webster, NY 14580 (US)
(72) Inventor: UNIDAD, Jerome, San Francisco, 94107 (US); COCKER, Eric, Redwood City, 94063 (US); MARTINI, Joerg, San Francisco, 94107 (US); IFTIME, Gabriel, Newark, 94560 (US); WEI, Junhua, Palo Alto, 94303 (US)
(74) Representative: Gill Jennings & Every LLP

(57) **Abstract**

Microorganisms present within a plurality of microorganism clusters immobilized in a porous support material may collectively define a supported bio-catalyst. When the microorganisms are effective to convert methane into one or more oxidized carbon compounds (e.g., methanotrophic bacteria), the supported bio-catalysts may be utilized to remove methane from methane-containing gas streams, such as those obtained from mining ventilation. Methods for processing a methane-containing gas stream may comprise interacting the gas stream with the supported bio-catalyst in substantial absence of a liquid phase, and obtaining a methane-depleted gas stream downstream from the supported bio-catalyst. Systems for processing a methane-containing gas stream may comprise the supported bio-catalysts housed in one or more vessels fluidly coupled to a source of methane-containing gas stream. A gas concentration in the methane-containing gas stream and/or the methane-depleted gas stream may be used to determine a current state or anticipated remaining lifetime of the supported bio-catalyst.

## Description

### FIELD

The present disclosure generally relates to removing methane from a gas stream.

### BACKGROUND

Methane and carbon dioxide are greenhouse gases that are produced in significant quantities in the U.S. and other countries. Although carbon dioxide is often produced in larger quantities than methane and receives significant media attention for its impact on global warming, methane is actually a stronger absorber of thermal energy than is carbon dioxide and represents an even more significant greenhouse gas concern. For example, over a period of at least 20 years, 1 ton of methane in the atmosphere contributes warming that is equivalent to approximately 24-36 tons of carbon dioxide. The most prevalent source of methane in the U.S. is the cattle industry, as a consequence of both ruminant digestion and manure degradation. Other significant methane sources include, for example, natural gas systems, landfills, coal mines and oil wells (both active and abandoned), and wastewater treatment facilities. Methane releases from many of these industries remain highly under-addressed.

Ventilation air from active coal mines represents the most significant source of released methane within the mining and petroleum industries. Mining ventilation reduces the underground methane concentration to a level below the explosive limit (5-15% v/v in air) and affords a gas stream having a low methane concentration (0.1-1% v/v) once expelled to the surface. Given the low methane concentrations and the high gas flow rates (50-500 m³/s) typically used to expel the exiting ventilation air, feasible methane abatement processes are limited. Consequently, diluted methane in the exiting ventilation air is usually released directly into the atmosphere. The released methane can represent a significant greenhouse gas concern. Moreover, if it could be properly harnessed, the released methane is a value product by itself or as a precursor to a number of value products.

Most methods for removing methane from a gas stream may require large spatial footprints and rely on oxidative processes (flaring or thermochemical conversion) to convert the methane into carbon dioxide. While such processes may be effective in industries producing more concentrated methane streams, they may be much less effective for gas streams having lower methane concentrations. In addition, flaring and undesirable atmospheric methane release both preclude conversion of the methane into more desirable value products. Current enzyme- and microorganism-based bioconversion approaches for methane abatement may address some of the foregoing difficulties, but they too presently require large spatial footprints as well as significant liquid contact with a bio-catalyst. Existing enzyme- and microorganism-based systems are also susceptible to inactivation by poisons, such as hydrogen sulfide, ammonia and carbon monoxide, which are frequently co-present with methane in various methane-containing gas streams. In addition, ongoing co-factor introduction may be required for maintaining bioactivity in some cases. High gas stream flow rates are also not particularly compatible with existing enzyme- and microorganism-based systems for promoting methane abatement. As such, there remains an unmet need for processing low-concentration methane-containing gas streams in a high-throughput manner, particularly through bio-catalysis, to preclude atmospheric release of this greenhouse gas.

Another difficulty associated with systems and methods for removing methane from various methane-containing gas streams is the need for frequent monitoring of the resulting methane-depleted gas streams to ensure that effective methane abatement has occurred. California Environmental Protection Agency requirements (Compliance Offset Protocol for Mine Methane Capture Projects-adopted 2014), for example, specify monitoring of methane fluxes passing through a removal or capture technology every two minutes. Monitoring may be conducted to determine, for example, if ineffective abatement has taken place, such as during a process upset, and/or if excessive poisons, such as carbon monoxide, ammonia, or hydrogen sulfide, are present in a methane-containing gas stream undergoing treatment. In addition, for carbon credit or carbon offset purposes, monitoring may determine if compliance with offset protocols has been met and/or whether a process may be certified as eligible for carbon credits or carbon offset. When utilizing a bio-catalyst to facilitate methane removal, ineffective methane abatement may occur, for example, if the bio-catalyst is nearing the end of its useful lifetime and/or if a poison (e.g., carbon monoxide, ammonia or hydrogen sulfide) has interfered with proper functioning of the bio-catalyst. Issues such as these are not typically present in flaring and thermochemical methane abatement processes, and they may often be monitored more easily. Without monitoring methane and other gas concentrations on an ongoing basis, it may be difficult to predict whether a bio-catalyst is apt to have exceeded its useful lifetime and/or to have become inactivated through poisoning. A further complicating difficulty is that effective sensing of methane and other gases may be problematic in low-concentration gas streams, such as those that are unable to be flared.

### SUMMARY

In some embodiments, the present disclosure provides supported bio-catalysts comprising: a porous support material; and a plurality of microorganism clusters immobilized within pores of the porous support material, one or more microorganisms within the microorganism clusters being effective to convert methane into one or more oxidized carbon compounds in substantial absence of a liquid phase. In some embodiments, the bio-catalysts may be present in a bio-catalyst module.

In other embodiments, the present disclosure provides methods for removing methane from a methane-containing gas stream. The methods comprise: interacting a methane-containing gas stream with a supported bio-catalyst in substantial absence of a liquid phase, the supported bio-catalyst comprising: a porous support material; and a plurality of microorganism clusters immobilized within pores of the porous support material, one or more microorganisms within the microorganism clusters being effective to convert methane into one or more oxidized carbon compounds; and obtaining a methane-depleted gas stream downstream from the supported bio-catalyst. Optionally, a concentration of one or more gases in the methane-containing gas stream and/or the methane-depleted gas stream may be determined at one or more monitoring locations.

In still other embodiments, the present disclosure provides systems for processing a methane-containing gas stream. The systems comprise: a gas inlet fluidly coupled to one or more vessels, the gas inlet providing a methane-containing gas stream to the one or more vessels and the one or more vessels housing a supported bio-catalyst comprising: a porous support material; and a plurality of microorganism clusters immobilized within pores of the porous support material, one or more microorganisms within the microorganism clusters being effective to convert methane into one or more oxidized carbon compounds in substantial absence of a liquid phase; and a gas outlet fluidly coupled to at least a portion of the one or more vessels and configured to remove a methane-depleted gas stream therefrom. Optionally, the systems may further comprise one or more monitoring locations for determining a concentration of one or more gases in the methane-containing gas stream and/or the methane-depleted gas stream, wherein a gas sensor is present at each of the one or more monitoring locations, the one or more monitoring locations are connected to a laser absorption spectroscopy system, or any combination thereof.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, as will occur to one having ordinary skill in the art and having the benefit of this disclosure.
FIG. 1 is a diagram of a first configuration of a bio-catalyst particle containing immobilized microorganism clusters.
FIG. 2 is a diagram of a second configuration of a bio-catalyst particle containing immobilized microorganism clusters.
FIG. 3A is a diagram of a system featuring a supported bio-catalyst of the present disclosure. FIG. 3B is a diagram of a system featuring a supported bio-catalyst of the present disclosure and incorporating a laser absorption spectroscopy system configured for measuring a gas concentration at one or more monitoring locations.
FIG. 4A is a diagram of the system in FIG. 3A coupled to ventilation air of a mine to provide a source of methane-containing gas stream. FIG. 4B is a diagram of the system of FIG. 3B coupled to ventilation air of a mine to provide a source of methane-containing gas stream.
FIG. 5A is a diagram of a system containing multiple vessels arranged in parallel, each vessel containing a supported bio-catalyst of the present disclosure. FIG. 5B is a diagram of a system containing multiple vessels arranged in parallel, each vessel containing a supported bio-catalyst of the present disclosure, and the system incorporating a laser absorption spectroscopy system configured for measuring a gas concentration at one or more monitoring locations.
FIG. 6A is a diagram of a system containing multiple vessels arranged in series, each vessel containing a supported bio-catalyst of the present disclosure. FIG. 6B is a diagram of a system containing multiple vessels arranged in series, each vessel containing a supported bio-catalyst of the present disclosure, and the system incorporating a laser absorption spectroscopy system configured for measuring a gas concentration at one or more monitoring locations.
FIG. 7 is a diagram of a system containing multiple vessels arranged in both series and parallel, each vessel containing a supported bio-catalyst of the present disclosure.
FIG. 8A is a diagram of a system containing multiple supported bio-catalyst modules housed in a single vessel. FIG. 8B is a diagram of a system containing multiple supported bio-catalyst modules housed in a single vessel, and the system incorporating a laser absorption spectroscopy system configured for measuring a gas concentration at one or more monitoring locations.
FIG. 9 is a diagram of a system in which oxygenated organic compounds may be drained from a vessel containing a supported bio-catalyst.
FIG. 10 is a diagram showing how onsite culturing and incorporation of methanotrophic microorganisms upon a porous support material may take place.
FIGS. 11-13 are process diagrams showing how methanotrophic microorganisms may be immobilized upon a porous support material.

### DETAILED DESCRIPTION

The present disclosure generally relates to methane removal from gas streams and, more particularly, bio-based catalysts, systems and methods effective to remove low-concentration methane from gas streams. The systems and methods may incorporate monitoring of a methane-containing gas stream and/or a methane-depleted gas stream to determine a concentration of one or more of methane, carbon dioxide, carbon monoxide, ammonia, hydrogen sulfide, or the like to determine methane abatement performance and to guide further methane abatement operations, such as for determining whether bio-catalyst replacement is warranted or if a process upset has occurred.

Although atmospheric release of methane is not easily avoidable in some industries (e.g., the cattle industry), there are several industries from which fairly localized methane-containing gas streams are obtained as significant contributors to greenhouse gas emissions. Mines and oil wells (both active and abandoned mines and wells), for example, are significant contributors to atmospheric methane release. Ventilation air from active mines, especially coal mines, may contain low concentrations of methane and be a predominant contributor to atmospheric release of methane. As discussed above, however, it may be problematic to remove low-concentration methane from a gas stream to preclude release of this greenhouse gas, particularly in a high-throughput manner. The low concentration of methane and other gases in a gas stream undergoing abatement may also complicate effective monitoring of a methane removal process.

The present disclosure provides bio-based systems and methods that may be effective for converting methane in various gas streams, including low-concentration methane-containing gas streams, into a less objectionable greenhouse gas *(i.e.,* carbon dioxide) and/or one or more value chemicals, particularly one or more oxygenated organic compounds. In particular, the present disclosure provides supported bio-catalysts comprising a plurality of microorganism clusters immobilized within pores of a porous support material. Methanotrophic bacteria, discussed in further detail below, may be a particularly effective type of microorganism for promoting methane removal from a gas stream in the disclosure herein. Suitable methanotrophic bacteria and other methanotrophic microorganisms may remain viable once immobilized within a porous support material and continue to be effective for converting methane into other substances. Advantageously, the supported bio-catalysts and related systems and methods disclosed herein may promote methane conversion in substantial absence of a liquid phase, thereby providing significant benefits over conventional bio-based methane conversion processes taking place in the presence of a bulk liquid phase, such as those in which a methane-containing gas stream is bubbled through the bulk liquid phase and at least partially solvated while contacting a bio-catalyst. Thus, the present disclosure may facilitate conversion of methane into one or more oxidized carbon compounds through a direct solid-gas interaction taking place in the absence of a bulk liquid phase.

Systems and methods incorporating the supported bio-catalysts disclosed herein may be particularly effective for converting methane in constrained gas streams (e.g., gas streams obtained from a discrete, localized source) having a low methane concentration, such as ventilation air from a mine. Such gas streams containing low-concentration methane may be difficult to process by conventional methane abatement techniques. The systems and methods described herein may be utilized to treat all or a significant portion of the ventilation air released from a particular mining locale and considerably decrease greenhouse gas emissions therefrom. If desired, carbon dioxide formed from methane may be subsequently processed by other carbon capture strategies, thereby decreasing overall greenhouse gas emissions still further. Alternately, the methane may be directly bio-converted into value organic chemicals, such as one or more oxygenated organic compounds, which may be further recovered, if desired. Such oxygenated organic compounds may include methanol, which may find use as a fuel, solvent or chemical precursor, and muconic acid, which is a precursor for valuable consumer bio-plastics such as nylon 66, polyurethanes or polyethylene terephthalate. The systems and methods of the present disclosure also offer advantages of modular design and operation and are discussed in further detail hereinafter. Moreover, coupled systems in which a methane-containing gas stream provides nutrients for methanotrophic microorganism culturing at a job site may provide still further advantages.

Systems and methods featuring the supported bio-catalysts disclosed herein may be advantageously implemented with minimal energy input other than during initial startup, given that methanotrophic microorganisms may perform their metabolic processes without receiving significant energy input. Recirculation of a methane-depleted gas stream to accomplish an even greater extent of methane removal may increase energy input requirements somewhat (e.g., energy needed to power fans, pumps, or the like) but provide advantages on the whole in terms of system-level efficiency gains beyond those provided by single-pass operations, thereby affording further methane abatement and lower greenhouse gas emissions.

Moreover, high loadings of microorganism clusters may be realized on a porous support material, thereby allowing high-throughput processing (e.g., at high flow rates) of methane-containing gas streams to take place. Extended lifetimes of the supported bio-catalysts may also be realized, with catalyst lifetimes of at least 2 months or more or even 6 months or more, such as about 2 months to about 8 months, being possible before replenishment or replacement of the supported bio-catalyst needs to take place. Microorganism loadings up to about 350 g/L may be realized upon the porous support materials disclosed herein, which is considerably higher than catalyst loadings possible with methane bioconversion strategies conventionally known in the art, particularly those featuring significant contact of a methane-containing gas stream with a bulk liquid phase. For example, conventional polymer matrices may afford loading values up to about 5 g/L, and hierarchically porous materials, such as fabricated carbon particles, may afford loading values up to only about 140 g/L. In addition to advantaged high-throughput processing offered by the embodiments disclosed herein, previously demonstrated strategies for removing methane from a gas stream using supported bio-catalysts may require significantly more operator intervention than with systems and methods of the present disclosure.

As a further advantage, the systems and methods of the present disclosure may further incorporate monitoring of an incoming methane-containing gas stream and/or a methane-depleted gas stream following methane abatement according to the disclosure herein. Monitoring may be conducted at a frequency sufficient to meet local, state or federal government regulatory requirements, such as every two minutes or less for strict regulatory standards. Frequent monitoring of an incoming methane-containing gas stream may offer alerts of potential process upsets that may occur due to the presence of poisons (e.g., excessive carbon monoxide, ammonia or hydrogen sulfide) and provide a baseline methane concentration for determining the effectiveness of methane removal following remediation. For example, a methane-depleted gas stream may be monitored at one or more monitoring locations to determine the effectiveness of the methane removal that has taken place in comparison to the baseline methane concentration. Poisons may be monitored in the methane-depleted gas stream as well, and, if present, their impact on the supported bio-catalyst may be determined. For example, a poison contacting the supported bio-catalyst at high concentration for a short period of time may be less detrimental than is a lower concentration exposure over an extended period of time. Accordingly, monitoring the methane-depleted gas stream may aid in determining, for example, 1) whether the supported bio-catalyst (or particular system modules containing the supported bio-catalyst) is functioning as intended or has reached the end of its effective lifetime and needs to be replaced, 2) the extent to which the methane-depleted gas stream needs to be recirculated (or recirculated to particular system modules containing the supported bio-catalyst), 3) the presence or effects of process upsets, such as occurring due to excessive poisons *(e.g.,* carbon monoxide, hydrogen sulfide, or ammonia), and how they have impacted the performance of the supported bio-catalyst, and 4) whether regulatory requirements have been met. Monitoring the methane-containing gas stream and/or the methane-depleted gas stream to observe compositional variations or potential process upsets may also trigger process modifications, either manual or automated, such as a reduction of incoming gas stream flow rates and/or recirculation flow rates, changes in system temperatures, and the like. Individual or multiple gas sensors may be utilized for these and other purposes to monitor an incoming methane-containing gas stream and/or a methane-depleted gas stream at a plurality of monitoring locations. Alternately or additionally, one or more common sensing channels capable of monitoring a gas sample received from multiple monitoring locations in a time-multiplexed manner may be incorporated in the methods and systems of the present disclosure, as explained in further detail hereinbelow. The one or more common sensing channels may be configured such that they are capable of detecting low concentrations of methane or other gases.

Terms used in the description and claims herein have their plain and ordinary meaning, except as modified by the paragraphs below.

As used herein, the term "room temperature" is about 25°C.

As used herein, the term "microorganism" refers to a single- or multi-celled entity of microscopic dimensions that is alive, and which may or may not be visible to the naked eye.

As used herein, the term "methanotrophic" refers to microorganisms that are methane-converting, particularly methane-oxidizing. Methanotrophic microorganisms may convert methane into one or more oxidized carbon compounds.

As used herein, the term "methylotrophic" refers to microorganisms that convert one-carbon compounds into products. Methanotrophic microorganisms are a subset of methylotrophic microorganisms.

As used herein, the term "bacteria" refers equivalently to prokaryotic bacteria, eukaryotic bacteria, eubacteria, and archaea. Methanotrophic bacteria can be any of these types.

As used herein, the term "microorganism cluster" refers to a plurality of microorganisms that are closely associated together and confined within a defined region. A bacterial colony is one type of microorganism cluster that may be used in the disclosure herein.

As used herein, the phrase "substantial absence of a liquid phase" means that a bulk liquid phase is not present. A bulk liquid phase is present when at least a portion of the microorganisms in a microorganism cluster are suspended, dispersed, or at least partially submerged in a liquid phase. A bulk liquid phase may include a liquid phase that may be removed by decantation, for example. Accordingly, "substantial absence of a liquid phase" encompasses embodiments in which the microorganism clusters are dry, exposed to water vapor (hydrated), or at least partially covered with a liquid film. A liquid film and similar constructs may not be removable by decantation until additional liquid is present to provide a bulk liquid phase.

Supported bio-catalysts suitable for use in the present disclosure may comprise a porous support material and a plurality of microorganism clusters immobilized within pores of the porous support material. One or more microorganisms within the microorganism clusters are effective to convert methane into one or more oxidized organic compounds in substantial absence of a liquid phase. In particular examples, the supported bio-catalysts may be effective to convert methane into one or more oxidized carbon compounds in substantial absence of a bulk aqueous phase, including a bulk water phase. Conditions encompassing substantial absence of a liquid phase, including substantial absence of a bulk aqueous phase, are provided above. In substantial absence of a liquid phase, sufficient gas exchange between a methane-containing gas stream and the microorganism clusters may occur for bioconversion of methane to take place, but without solvation of gas taking in a bulk liquid phase occurring.

In particular examples, the microorganism clusters may comprise at least one type of methanotrophic bacteria. Methylotrophic bacteria may be used similarly in some instances. Suitable methanotrophic bacteria may be effective to convert methane into one or more oxidized organic compounds under aerobic conditions but are otherwise not believed to be particularly limited. Alternately, anaerobic conversion of methane may occur suitably in some instances. Thus, methanotrophic bacteria of any of Type I, Type II, Type X, or any combination thereof may be utilized in the disclosure herein. *Methylotuvimicrobium alcaliphilum sp. 20Z* is one suitable example of a type of methanotrophic bacteria that may be utilized in the various embodiments of the present disclosure. *M. modestohalophilus 10S* may also be used in a similar manner within a supported bio-catalyst used for processing a methane-containing gas stream according to the disclosure herein. Other bacteria species that may be used in the disclosure herein include, for example, *Methylocella tundra,* which are facultative methanotrophs and can synthesize methanol; aerobic Type I methanotrophs such as species belonging to the following genera: *Methylomonas, Methylobacter, Methylosarcina, Methylosoma* and *Methylococcus;* and aerobic Type II methanotrophs such as *Methylocapsa aurea* and species belonging to the genera *Methylocystis* and *Methylosinus.* Bacteria belonging to the category of halophilic methanotrophs such as *M. alcaliphilum sp. 20Z* and *M*. *modestohalophilus 10S*, among others, are of interest given their robustness, survival under extreme conditions, and temperature tolerance up to about 70°C.

In addition to methanotrophic bacteria, other types of microorganisms capable of converting methane into one or more oxidized carbon compounds may also be suitable for use in the disclosure herein. For example, methanotrophic fungi may also be suitable, such as *Graphium sp.,* as well as methylotrophic yeasts such as *Pichia pastoris,* other *Pichia sp., Candida sp., Torulopsis sp. and Hansenula sp.*

Any of native (wild type) or engineered methanotrophic or methylotrophic bacteria or other microorganisms may be used in accordance with the disclosure herein. Wild type or engineered methanotrophic bacteria or other microorganisms may convert methane into carbon dioxide when interacted with a methane-containing gas stream. Engineered methanotrophic bacteria or microorganisms producing carbon dioxide from methane may also be genetically modified to produce lower amounts of side product or poisons, have enhanced tolerance to poisons (e.g., carbon monoxide, ammonia, higher alkanes, or hydrogen sulfide), produce higher yields of carbon dioxide or other oxidized carbon compounds per unit amount of methane, or any combination thereof. In some or other embodiments of the present disclosure, engineered methanotrophic bacteria may convert methane into one or more oxygenated organic compounds, such as methanol or even larger oxygenated organic compounds, such as muconic acid, which may be recovered as value chemicals, if desired. Methanol produced in accordance with the disclosure herein may, for example, be formulated within biofuels or be refined as a solvent or chemical precursor. Muconic acid is a high-value chemical, since it may be used as a precursor to bioplastics such as polyethylene terephthalate, polyurethanes and nylon 66. The value chemicals produced in accordance with the foregoing may be leeched continuously from the supported bio-catalyst or a vessel (or module) housing the supported bio-catalyst, or the value chemicals may be recovered from the supported bio-catalyst periodically (e.g., during catalyst replenishment or replacement). In some instances, it may be necessary to rupture and sacrifice the microorganisms to recover oxygenated organic compounds contained therein, which may be more desirably conducted during replacement of the supported bio-catalyst to minimize process downtime. In some instances, monitoring the methane-depleted gas stream according to aspects of the disclosure herein may provide further guidance as to whether recovery of oxygenated organic compounds from the supported bio-catalysts may be desirable prior to a regular cycle of catalyst replenishment and replacement.

Microorganism clusters suitable for use in the disclosure herein may contain any number of cells, provided that the microorganism clusters may be successfully housed and retained within the pores of the porous support material. That is, a maximum size of the microorganism clusters may be no larger than the pores in the porous support material in which they are immobilized. In illustrative examples, the microorganism clusters may contain about 2 to about 50 cells, or about 5 to about 25 cells, or about 6 to about 15 cells, or about 7 to about 11 cells, or about 8 to about 10 cells. In various embodiments, the largest dimension of the microorganism clusters may range from about 2 microns to about 100 microns, or about 5 microns to about 80 microns, up to the maximum size of the pores of the porous support material. The cells within the microorganism clusters may remain closely associated with each other once the microorganism clusters have been incorporated within the porous support material. Techniques for making microorganism clusters are not believed to be particularly limited and are known in the art. Illustrative techniques may include, for example, dispersing a plurality of microorganisms in a liquid phase, applying mechanical force (e.g., stirring, agitation, vibration, sonication, or the like), performing microfluidic techniques (e.g., cell encapsulation by droplet microfluidics), spraying cell-laden suspensions, ink-jetting cell-laden suspensions, optionally including a surfactant or dispersant, optionally performing phase inversion, and/or promoting emulsion formation. The liquid phase may be at least partially removed to collect the microorganism clusters.

The microorganism clusters, such as clusters of methanotrophic bacteria, may be introduced to the pores of a porous support material after the porous support material has been formed. Alternately, the methanotrophic microorganisms may be present while the porous support material is being formed, such that the porous support material forms around the microorganism clusters. The size of the microorganism clusters may dictate to some degree the pore sizes that are obtained in this instance. Suitable porous support materials may include morphological constructs such as, for example, aerogel scaffolds, hydrogel scaffolds, sol-gel scaffolds, cellulose scaffolds, hierarchically porous activated carbon scaffolds, large-pore zeolite scaffolds, metal-organic framework scaffolds, polymer composite scaffolds, and porous polymer scaffolds, including porous epoxy and other thermoset scaffolds, and the like. Any porous material may be utilized to provide the porous support material in the disclosure herein, provided that the porous material can successfully immobilize the microorganism clusters therein while also maintaining the microorganisms in a viable state.

In some examples, suitable porous support materials may include porous polymer supports containing particles, such as those described in U.S. Patent Application Publication 2019/0241883, which is incorporated herein by reference in its entirety. Such porous support materials may be formed by polymerizing a plurality of polymerizable monomers in the presence of a plurality of particles. Once formed, clusters of methanotrophic microorganisms, such as methanotrophic bacteria, may be introduced into the pores of the porous support material, such as through liquid infiltration. Pore sizes may range from about 200 nm to about 5 microns. Suitable particles that may be included in the porous support materials include, but are not limited to, amorphous carbon, carbon black, silica, alumina, graphene, graphite, carbon nanotubes, metal particles or flakes, metal nanoparticles, silicon carbide, aluminum nitride, cellulose nanocrystals, clay platelets, chopped or milled carbon fibers, and the like. Suitable particle shapes may include, for example, spheres, cylinders, plates, fibers, irregular shapes, beads, and the like.

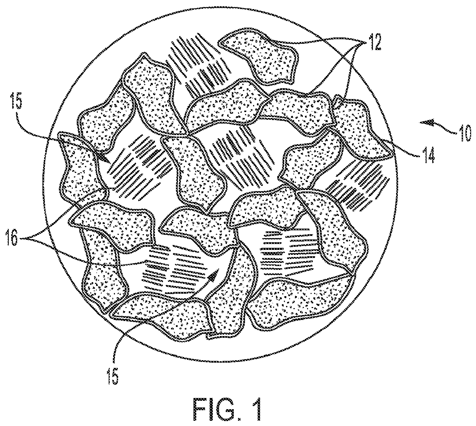

FIG. 1 is a diagram of a first configuration of a bio-catalyst particle containing immobilized microorganism clusters. As shown, bio-catalyst particle 10 includes particles 12, each having polymer coating 14 thereon. Polymer coating 14 binds adjacent particles 12 together and defines pores 15 in between. Within at least a portion of pores 15 are immobilized microorganism clusters 16, particularly clusters of methanotrophic bacteria and/or methylotrophic fungi. Polymer coating 14 may be formed upon particles 12 through an *in situ* polymerization process in which particles are present in a polymerizable monomer. Following *in situ* polymerization, a porous support lacking microorganism clusters 16 may be obtained. Once a porous support has been obtained, microorganism clusters 16 may be introduced via liquid infiltration into pores 15.

Alternately, microorganism clusters 16 may be present as the porous support is being formed via *in situ* polymerization, in which case microorganism clusters 16 may be directly incorporated into pores 15 without a separate liquid infiltration process taking place.

Polymerizable monomers that may be utilized to form suitable bio-catalyst particles 10 in the foregoing manner are not believed to be particularly limited. In non-limiting examples, suitable monomers may be ethylenically unsaturated monomers that are polymerizable in the presence of a radical initiator or photoinitiator. Examples of such monomers may include, but are not limited to, vinyl, acrylate, and methacrylate monomers. Illustrative examples of suitable acrylates and methacrylates include (a) monofunctional acrylates and methacrylates such as methyl acrylate, ethyl acrylate, methyl methacrylate, ethyl methacrylate, benzyl methacrylate, lauryl methacrylate, isobornyl methacrylate, (b) difunctional acrylates and methacrylates such as 1,3-butanediol diacrylate, 1,6-hexanediol diacrylate, bisphenol A ethoxylate diacrylate, ethylene glycol diacrylate, poly(ethylene glycol) diacrylate, (c) tri-, tetra-, penta- or hexa-acrylates and methacrylates such as trimethylolpropane triacrylate, trimethylolpropane ethoxylate triacrylate, di(trimethylolpropane) tetraacrylate, dipentaerythritol pentaacrylate, hexaacrylate, and the like. Suitable vinyl monomers include styrene, divinylbenzene and liquid ethylene derivatives such as, vinyl stearate, vinyl laurate, vinyl benzoate, vinyl acetate, ethyl vinyl ether, vinyl chloride, and 1-vinyl-2-pyrrolidone.

Examples of radical initiators include thermal initiators (activated by heat) and photoinitiators (activated by light), typically ultraviolet light in a range of about 200 nm to 400 nm wavelength. Non-limiting examples of thermal initiators include (a) peroxides such as benzoyl peroxide, diacetylperoxide, di t-butylperoxide, or cumyl peroxide; or (b) azo compounds such as AIBN and phenylazotriphenylmethane. Non-limiting examples of photoinitiators include benzophenone, benzil, benzoin and the like.

Polymerization may take place in water or a water-organic solvent mixture. In addition to water, other solvents may include mixtures of water with other compatible solvents, such as ethanol, propanol or butanol. The ratio of the other solvent to water may range from 0.1:99.9 to 90:10 on a volume basis.

In another example, the supported bio-catalysts may be produced by mixing particles and microorganism clusters in water with a small amount of polymer, followed by optional solvent evaporation. This process may create a scaffold in which the polymer forms a coating and holds the particles together. The amount of polymer may be chosen such that a thin layer of dry polymer is formed after evaporation and acts as a binder to keep the particles together and maintain structural integrity of the supported bio-catalyst. Suitable polymers include those that are soluble in water or mixtures of water with other solvents. Examples of suitable polymers include polyvinylpyrrolidone, polyvinyl alcohol, polyethylene glycols, polyacrylamides, polyethyleneoxide, poly 2-hydroxyethyl methacrylate, hydroxypropyl methylcellulose, hydroxyethyl cellulose, other modified celluloses, and chitosan.

When the supported bio-catalysts are formed by mixing particles with a polymeric binder and microorganism clusters in solution, various parameters such as temperature may be modulated to tune the resulting structural morphology. Prior to drying, parameters such as the temperature and pH of the solvent may be changed to force self-assembly or structure formation between the polymer and the particles. For example, the temperature may be elevated or reduced relative to room temperature, where a self-assembled structure forms around the microorganism clusters. Morphologies obtained prior to drying may look similar to particle-stabilized emulsions where the particles are acting as a surfactant or dispersant and at least partially preclude aggregation of the microorganism clusters. During drying, the temperature and evaporation rates may also be modulated to control the resulting microstructure of the porous support.

Copolymers made of more hydrophobic polymers and hydrophilic polymer segments are also suitable in the foregoing. Some examples include poly(acrylic acid-co-styrene), acetate-ethylene copolymer, ethylene vinyl acetate, polyethylene glycol-polycaprolactone, and polyethylene glycol-polyethyleneimine. This approach may result in incorporation of large amounts of particles into the porous support material, giving it a high particle to polymer ratio. The amount of polymer with respect to the amount of particles is therefore small in this case. The weight/weight ratio between the polymer and particles may range from about 0.1% to about 50% on a weight basis. This approach makes the porous support material particularly suited for processes that take place in the absence of water, or that use only small amounts of water, and where fast heat removal is desirable, particularly when highly thermally conductive particles are used. Ready heat dissipation may also aid in preventing the microorganisms from becoming inactivated during the process of converting the methane to oxidized carbon compounds. Thus, in at least some embodiments, the supported bio-catalysts disclosed herein may comprise a porous support material that comprises a plurality of thermally conductive particles.

In some examples, suitable porous support materials may include porous polymer supports, optionally lacking embedded particles, such as those described in U.S. Patent Application Publication 2020/0189177, which is incorporated herein by reference in its entirety. Such porous support materials may be produced by forming a mixture of solvent and polymerizable monomers in a desired shape, polymerizing the polymerizable monomers to form a polymerized mass in the presence of the solvent, and lyophilizing or applying vacuum to remove the solvent and leave behind a plurality of pores in the polymerized mass. Optionally, preformed polymer may be dissolved in a solvent and shaped without an additional polymerization step taking place. Once formed, clusters of methanotrophic microorganisms, such as methanotrophic bacteria, may be introduced into the pores of the porous support material, such as through liquid infiltration. Pore sizes may be adjusted based upon the amount of solvent that is present and range from about 200 nm to about 5 microns. Prior to solvent removal to define pores, the mixture of solvent and polymerizable monomers (or pre-formed polymer) may be deposited in a desired shape via 3-D printing or casting, for example.

The solvent may comprise about 50% or greater of the mixture of solvent and polymerizable monomers (or pre-formed polymer) on a mass basis. Suitable solvents may have a melting temperature of about -20°C or greater, such that they are liquids at room temperature. Non-limiting examples of suitable solvents may include, but are not limited to, one or more of 1-octadecanol, water, diethylene glycol, triethylene glycol, tetraethylene glycol, decane, n-decanol, propylene glycol methyl ether acetate, ethyl-3-ethoxy propionate, 2-heptanone, and 2,3-dimethyl-4-heptanone. According to some implementations, the solvent may comprise a crystalline molecule that melts at a temperature of about 10°C or more above the ambient temperature at which the mixture of solvent and polymerizable monomers is shaped *(e.g.,* by 3-D printing or casting).

The polymerizable monomers (or pre-formed polymer) may be soluble in the solvent and form a thermoset material once polymerized. The thermoset material may be combined with a thermoplastic material in some instances. Suitable thermoset materials/polymerizable monomers may include one or more of epoxy resin, polyester resin, polyurethanes, vulcanizable rubber, polyimides, silicone, and vinyl ester, for example. Suitable thermoplastic materials that may be combined with a thermoset material include, but are not limited to, one or more of a polymer comprising lactide and glycolide, chitosan, hydroxybutyric acid, polyanhydrides, polyesters, polyphosphazenes, polyphosphoester, caprolactone polymer, alginate, agar, polyurethane, and/or gelatin.

The polymerized mass may additionally comprise fillers and/or nanoparticles. In some implementations, the filler may comprise polymer particles or polymer nanoparticles. Thus, a thermoplastic polymer admixed with the polymerized mass may be distributed discontinuously in particle form, according to some embodiments. Other suitable particles that may be present within the polymerized mass include, but are not limited to, amorphous carbon, carbon black, silica, alumina, graphene, graphite, carbon nanotubes, metal particles or flakes, metal nanoparticles, silicon carbide, aluminum nitride, cellulose nanocrystals, clay platelets, chopped or milled carbon fibers, and the like.

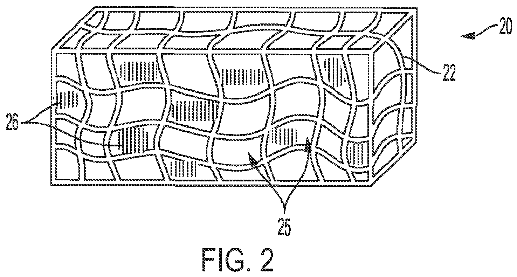

FIG. 2 is a diagram of a second configuration of a bio-catalyst particle containing immobilized microorganism clusters. As shown, bio-catalyst particle 20 includes porous scaffold 22 containing pores 25 defined therein. Alternately, bio-catalyst particle 20 may constitute a larger structure, such as a slab or monolith having a similar microstructure. Within at least a portion of pores 25 are immobilized microorganism clusters 26, particularly clusters of methanotrophic bacteria. Once a porous support has been obtained, microorganism clusters 26 may be introduced via liquid infiltration into pores 25. Alternately, microorganism clusters 26 may be present as porous scaffold 22 is being formed. Bio-catalyst particle 20 differs from bio-catalyst particle 10 (FIG. 1) in that porous scaffold 22 is a continuous structure in bio-catalyst particle 20, whereas there are grain boundaries between particles 12 and their polymer coating 14 in bio-catalyst particle 10 (FIG. 1). Porous scaffold 22 may or may not contain a plurality of discrete particles (not shown) within the continuous structure defined therein.

Accordingly, the supported bio-catalysts described herein may be present in a powder form, a slab form, and/or be further containerized within a cartridge or housing, for example. Containerization of the supported bio-catalysts within a cartridge or housing may permit modular implementation of the supported bio-catalysts to be realized, as described in further detail hereinafter. Slab forms may be fabricated by casting as a porous monolith. Slab forms may be particularly desirable for modular implementation of the supported bio-catalysts to be realized. It is to be appreciated that powder forms of the supported bio-catalysts may also be implemented modularly in the disclosure herein.

Once clusters of methanotrophic microorganisms have been suitably immobilized within a porous support material, a methane-containing gas stream may be interacted with the supported bio-catalyst, particularly a plurality of particles comprising the supported bio-catalyst, optionally in modular form. After the gas stream has suitably interacted with the supported bio-catalyst, a methane-depleted gas stream may be obtained downstream from the supported bio-catalyst. Carbon dioxide within the methane-depleted gas stream may be further sequestered using an appropriate carbon capture technology, if desired. The carbon dioxide may originate from the methane-containing gas stream itself and/or be produced by bio-conversion of methane by the methanotrophic microorganisms. If produced, oxygenated organic compounds may be recovered from the methanotrophic microorganisms and/or a vessel or module housing the supported bio-catalyst. Alternately, the methane-depleted gas stream may undergo atmospheric release following production thereof and/or recirculation of at least a portion of the methane-depleted gas stream may take place. Monitoring of the methane-depleted gas stream may also take place using one or more appropriately placed sensors, which may provide documentation that local environmental regulations are being met, for example. Monitoring of the methane-depleted gas stream and/or the methane-containing gas stream may provide additional information that may facilitate methane abatement according to the disclosure herein, as discussed further hereinbelow.

Accordingly, methods for processing a methane-containing gas stream according to the present disclosure may comprise: interacting a methane-containing gas stream with a supported bio-catalyst in substantial absence of a liquid phase, the supported bio-catalyst comprising: a porous support material; and a plurality of microorganism clusters immobilized within pores of the porous support material, one or more microorganisms within the microorganism clusters being effective to convert methane into one or more oxidized carbon compounds; and obtaining a methane-depleted gas stream downstream from the supported bio-catalyst. The flow rate and flux of the methane-containing gas stream across the supported bio-catalyst may be maintained at levels to support a desired rate of methane conversion. Optionally, at least a portion of the methane-depleted gas stream may be recirculated to the supported bio-catalyst. In addition, or further optionally, the methane-containing gas stream and/or the methane-depleted gas stream may be analyzed in-line using one or more sensors to determine a concentration of methane, carbon dioxide, carbon monoxide, ammonia, hydrogen sulfide, or any combination thereof. A plurality of sensors may be utilized to determine the concentration of a particular gas at a specified monitoring location, or an in-common sensing channel may receive and analyze a gas sample from a plurality of monitoring locations in a time-multiplexed manner. All gases subject to monitoring need not necessarily be monitored at each monitoring location. Further details regarding suitable monitoring locations and in-common sensing channels are provided in additional detail hereinbelow.

The methane-depleted gas stream may also be provided to non-biological carbon capture processes, such as a regenerative thermal oxidation process, if desired. For example, a carbon capture process capable of sequestering carbon dioxide may be utilized for further processing the methane-depleted gas stream in some instances.

Optionally, a nutrient source may be provided to the methanotrophic microorganisms, such as methanotrophic bacteria, prior to interacting the methane-containing gas stream with the supported bio-catalyst. For example, a nutrient source may be provided during storage or while transferring the supported bio-catalysts to a job site. The nutrient source may be a liquid, such as methanol. Additional suitable nutrient liquids may include, for example, liquid alkanes, other alcohols, and various other suitable carbon-containing compounds. Optionally, methane gas may be provided to the supported bio-catalysts as a nutrient source for the methanotrophic microorganisms. The nutrient source for the methanotrophic microorganisms, such as methanotrophic bacteria, may reside within the pores of the porous support material when appropriately provided thereto. The nutrient source may be removed or consumed prior to the supported bio-catalysts being interacted with a methane-containing gas stream. Optionally, a portion of a methane-containing gas stream may provide methane as a nutrient source for methanotrophic microorganisms before conducting methane abatement of the methane-containing gas stream in accordance with the disclosure herein.

Gas streams suitable for use in the disclosure herein may contain an amount of methane that is insufficient for flaring or other common methane removal techniques. In various embodiments, suitable gas streams may comprise about 20% methane by volume or less *(i.e.,* a practical lower methane concentration limit effective for flaring), or about 5% methane by volume or less *(i.e.,* a practical lower concentration limit of combustion). The methane-containing gas stream may comprise any non-zero amount of methane up to these levels. In particular examples, the methane-containing gas stream may comprise ventilation air from an operating mine *(e.g.,* a coal mine) or outflow air from an inactive mine. Ventilation from such mines may be described as "gassy" *(i.e.,* producing high methane concentrations in enriched air), in which ventilated gas comprises about 0.1-1% methane by volume, or "non-gassy," in which ventilated gas can have an even lower methane concentration (down to about 0.04% by volume). Alternative sources of methane-containing gas stream may include any other origin from which a coherent flow of methane-containing gas may be obtained and provided to the supported bio-catalyst. Other suitable sources of methane-containing gas streams comprising low levels of methane include, but are not limited to, oil and gas wells, natural gas processing facilities, landfills, chemical processing facilities, refineries producing low levels of methane byproducts, manure management from agricultural farms, factory farms (e.g., methane produced from enteric fermentation), wastewater treatment facilities and the like. Other than the methane concentration, the methane-containing gas streams obtained from the foregoing sources may also be characterized by their low volumetric flow rates, which are about 50-500 m³/s for actively vented coal mines and significantly lower for abandoned coal mines or gas wells (about 200-375 mcfd or even thousands of cubic feet per day). Lower flow rates can also be expected from landfills, which have flow rates that may vary from about 600-1250 scfm under wet conditions to about 400-600 scfm under dry conditions. Accordingly, the methane-containing gas stream or a recirculated methane-depleted gas stream may be supplied to the supported bio-catalysts of the present disclosure at a flow rate of about 50 scfm to about 1250 scfm, or about 50 scfm to about 500 scfm, or about 400 scfm to about 600 scfm, or about 1200 scfm. Gas flow rates below 50 scfm are also possible in the disclosure herein, as are gas flow rates above about 1200 scfm. For example, some system configurations may be compatible with gas flow rates ranging from about 50000 to about 100000 scfm.

It is to be appreciated that gas streams containing higher levels of methane may also be processed in accordance with the disclosure herein. Higher methane concentrations in the methane-containing gas stream may be desirable if an oxygenated organic compound is being produced as a value chemical for recovery. That is, higher methane concentrations may be especially desirable to utilize when the supported bio-catalysts are being used to conduct chemical synthesis. At higher methane concentrations in the methane-containing gas stream, heat dissipation from the supported bio-catalysts may become an increasing concern. Heat dissipation may be alleviated, at least to some degree, by utilizing porous support materials incorporating particles having a high thermal conductivity. Active cooling (e.g., with a chilled water or room temperature water system in thermal communication with the supported bio-catalyst) may also be utilized if excessive heating occurs. It is to be appreciated that use of the supported bio-catalysts in higher concentrations of methane may be favorable for improving the viability of the methanotrophic or methylotrophic microorganisms. Given the abundance of methane in such gas streams, the microorganism viability may reach 6 months or more, versus about 2-6 months of viability in lower concentrations of methane expected from mine ventilation and other low-concentration methane sources. A determination of microorganism viability in the supported bio-catalysts may be further aided by sensing the methane concentration in the methane-containing gas stream and/or the methane-depleted gas stream, as described further herein.

Optionally, air or oxygen may be introduced to the methane-containing gas stream (or supplied directly to the supported bio-catalyst in a reactor or similar vessel housing the supported bio-catalyst). Introduction of additional air or oxygen to the methane-containing gas stream or the supported bio-catalyst may be conducted if there is insufficient oxygen for the methanotrophic microorganisms to support aerobic conversion of the methane into oxygenated organic compounds or carbon dioxide.

The supported bio-catalysts may be present within a reactor or similar vessel in various forms, such as powders, sheets, slabs, beads, or defined lattice structures. The form of the supported bio-catalysts is not believed to be particularly limited, provided that the form can allow a sufficient flux of methane-containing gas stream to pass therethrough.

After passing through supported the bio-catalyst, the methane-depleted gas stream may experience a decrease in methane concentration of about 80% or more, such as a decrease in methane concentration of about 80% to about 95%, or even a decrease up to about 99%, as compared to the methane concentration in the methane-containing gas stream. That is, about 80% to about 95%, or even up to about 99%, of the methane in the methane-containing gas stream supplied to the supported bio-catalyst may be converted to carbon dioxide or an oxygenated organic compound. These methane concentrations may be accomplished following recirculation of at least a portion of the methane-depleted gas stream in some cases, should initial passage of the methane-containing gas stream not result in a desired amount of methane removal. Alternately, the methane-depleted gas stream may contain only about 5% to about 20% of the methane concentration present in the methane-containing gas stream provided to the supported bio-catalyst. Recirculation of at least a portion of the methane-depleted gas stream may be conducted to achieve higher methane conversion or abatement, if desired, especially if less effective methanotrophic microorganisms are used or if local regulatory requirements are particularly stringent, for example. When less effective methanotrophic microorganisms are used, the decrease in methane concentration can be as low as 50% or as high as 80%, but may be increased by recirculation of at least a portion of the methane-depleted gas stream, if desired. Lower conversion may be favorable if the cost of the methanotrophic microorganisms is a concern or if less effective bacteria or fungi are easier to produce and/or use. Recirculation rates of the methane-depleted gas stream may range from 0% (no recirculation, 100% overall release of the methane-containing gas stream following abatement) to about 95% (10% overall release of the methane-containing gas stream following abatement). A determination of whether recirculation should occur and the extent to which recirculation occurs may be further aided by sensing the methane concentration in the methane-containing gas stream and/or the methane-depleted gas stream, as described further herein.

The supported bio-catalysts of the present disclosure may be implemented in systems having various configurations that may support application-specific needs. In various examples, systems of the present disclosure may comprise: a gas inlet fluidly coupled to one or more vessels, the gas inlet providing a methane-containing gas stream to the one or more vessels and the one or more vessels housing a supported bio-catalyst comprising: a porous support material; and a plurality of microorganism clusters immobilized within pores of the porous support material, one or more microorganisms within the microorganism clusters being effective to convert methane into one or more oxidized carbon compounds in substantial absence of a liquid phase; and a gas outlet fluidly coupled to at least a portion of the one or more vessels and configured to remove a methane-depleted gas stream therefrom. Optionally, the systems may be configured to recirculate at least a portion of a methane-depleted gas stream to the supported bio-catalyst. In addition, or further optionally, the systems may further comprise one or more monitoring locations for determining a concentration of one or more gases in the methane-containing gas stream, the methane-depleted gas stream, or any combination thereof. At the one or more monitoring locations, there may be one or more gas sensors *(e.g.,* a gas sensor present at each monitoring location), the one or more monitoring locations may be connected to a laser absorption spectroscopy system, or any combination thereof. The one or more gas sensors and/or the laser absorption spectroscopy system may be effective to determine, for example, a concentration of methane, carbon dioxide, carbon monoxide, ammonia, hydrogen sulfide, or any combination thereof in the methane-containing gas stream and/or the methane-depleted gas stream at the various monitoring locations. A plurality of gas sensors may be utilized to determine the concentration of a particular gas at a specified location or multiple locations, or an in-common sensing channel may receive and analyze methane-containing gas stream and/or methane-depleted gas stream from a plurality of locations and analyze the gas concentration therein in a time-multiplexed manner, as described further herein.

In the disclosure herein, any gas sensor or sensing system capable of detecting and/or quantifying, for example, methane, carbon dioxide, ammonia, hydrogen sulfide, and/or carbon monoxide may be utilized. Suitable sensors may include any one or a combination of optical sensors (e.g., infrared and near-infrared sensors), calorimetric sensors, pyroelectric sensors, semiconducting metal oxide sensors, electrochemical sensors, capacitive sensors, resistive sensors (including those incorporating functionalized nanomaterials, such as carbon nanotubes), and the like. Suitable gas sensors may be capable of detecting these gases down to a detection limit of about 0.1 wt. %, or about 0.01 wt. %, or even about 0.001 wt. % (10 ppm).

In some embodiments, suitable sensors may comprise printed electronic sensors of the "peel and stick" kind that are low-cost and may be deployed in multiple locations. Such sensors typically operate based upon resistive or capacitive principles when detecting methane by adsorption. Absorption onto specially functionalized inks may modify the resistance or the capacitance of the deposited ink, which may then be measured electrically.

One suitable type of sensor may include a tunable diode laser absorption spectroscopy system. Such sensors may comprise a diode laser source providing electromagnetic radiation transiting a path length along which a gas stream of interest is located, and a detector configured to receive the electromagnetic radiation after interacting with the gas stream of interest while transiting the path length. The detector may be capable of detecting a single wavelength or multiple wavelengths, such as may be performed with a diode array detector. A diode array detector may be utilized to analyze for multiple gases simultaneously, or two different wavelengths of electromagnetic radiation received upon interacting with a single gas. To account for the low gas concentrations that may be present in the gas stream undergoing analysis, the path length may be made sufficiently long to provide a desired level of detection. In various embodiments, the path length suitable for use in detecting methane, carbon dioxide, ammonia, hydrogen sulfide, and/or carbon monoxide in the disclosure herein may be about 0.1 m or greater, or about 0.5 m or greater, or about 1 m or greater, or about 2 m or greater, or about 5 m or greater, or about 10 m or greater, or about 20 m or greater. In more specific examples, the path length may range from about 1 m to about 25 m, or about 5 m to about 20 m, or about 10 m to about 20 m.

A tunable diode laser absorption spectroscopy system may be present at one or more locations where methane, carbon dioxide, ammonia, hydrogen sulfide, and/or carbon monoxide sensing is needed. However, a single, in-common tunable diode laser absorption spectroscopy system may be present to afford sensing capabilities at one or more monitoring locations within systems containing the supported bio-catalysts of the present disclosure. Such systems may contain one or more lines configured to supply a gas side stream (gas sample) from a first monitoring location to the flow pathway of the tunable diode laser absorption spectroscopy system in a time-multiplexed manner, and after a gas analysis has taken place, the flow pathway may then be vented in preparation for receiving a gas side stream (gas sample) from a second monitoring location. The systems may cycle through each or at least a portion of the available monitoring locations over a cycle time compatible with gas detection at a sufficient accuracy and with sufficient frequency to satisfy regulatory requirements in a given locale or other process requirements (e.g., to facilitate active process control). In some embodiments, a first tunable diode laser absorption spectroscopy system may be present for detecting methane and carbon dioxide, which may be performed simultaneously or near simultaneously, and a second tunable diode laser spectroscopy system may be present to detect carbon monoxide in a specified location. Alternately, a tunable diode laser absorption spectroscopy system may comprise a first laser suitable capable of analyzing a first gas or mixture of first gases (e.g., methane and/or carbon dioxide) and a second laser different than the first laser and capable of analyzing a second gas or mixture of second gases (e.g., carbon monoxide, ammonia, and/or hydrogen sulfide). As still another alternative, a tunable diode laser absorption spectroscopy system may be utilized in combination with a carbon monoxide sensor operating in a different manner than does the tunable diode laser absorption spectroscopy system, such as an electronic gas contact sensor effective for detecting carbon monoxide, including those operating based on electrical resistance principles, capacitive principles, optical detection, heat-based detection, or calorimetric detection, among others. Such sensors for detecting carbon monoxide may include those capable of detecting carbon monoxide down to a detection limit of about 1.5 ppm, for example. In some examples, a tunable diode laser absorption spectroscopy system, preferably a single tunable diode laser spectroscopy system, may be configured to analyze for methane and/or carbon dioxide received from a plurality of monitoring locations in a time-multiplexed manner, and a sensor configured for monitoring carbon monoxide may be present at each of the monitoring locations.

It is to be further noted that the systems and methods of the present disclosure do not need to monitor all of methane, carbon dioxide, and carbon monoxide at each potential monitoring location. Indeed, some monitoring locations may monitor each of these gases, while others may monitor only one of these gases or only two of these gases. In some instances, methane may be monitored at each potential monitoring location, and carbon dioxide and/or carbon monoxide may be monitored optionally in combination with methane. As discussed above, monitoring of methane (and optionally carbon dioxide) may take place using a diode laser absorption spectroscopy system.

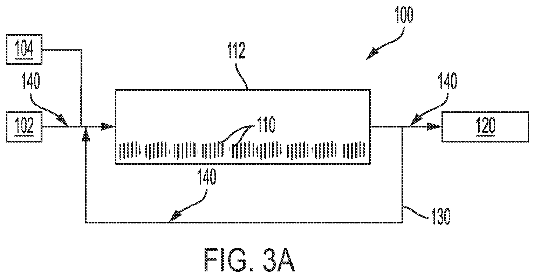

Various system configurations are described hereinafter in reference to FIGS. 3A, 3B, 4A, 4B, 5A, 5B, 6A, 6B, 7, 8A, 8B, 9, and 10. Common reference characters are used to denote elements having similar structure and function in multiple figures. Each of the system configurations shown in the foregoing FIGS. may optionally incorporate monitoring capabilities for methane, carbon dioxide, ammonia, hydrogen sulfide, and/or carbon monoxide at one or more specified monitoring locations. It should be appreciated that the depicted monitoring locations in these FIGS. are exemplary, and monitoring need not necessarily take place at every depicted monitoring location, nor does monitoring of each of methane, carbon dioxide, ammonia, hydrogen sulfide, and carbon monoxide need to take place at each depicted monitoring location. Moreover, monitoring may take place at additional monitoring locations beyond those that are specifically depicted in the drawings.

FIG. 3A is a diagram of a system featuring a supported bio-catalyst of the present disclosure. As shown in FIG. 3A, system 100 includes methane-containing gas stream 102 that is supplied to supported bio-catalyst 110 housed within vessel 112. Optionally, oxygen or air 104 may be supplied to methane-containing gas stream 102 prior to or while entering vessel 112, or oxygen or air 104 may be supplied directly to vessel 112. Oxygen or air 104 may be added if sufficient oxygen is not present to support effective bioconversion of methane with vessel 112. That is, if methane-containing gas stream 102 does not provide sufficiently aerobic conditions to facilitate biocatalysis, additional oxygen or air 104 may be added. After interacting with supported bio-catalyst 110, methane-depleted gas stream 120 is obtained downstream from vessel 102.

Carbon dioxide formed through bioconversion of methane within vessel 112 may be present in methane-depleted gas stream 120 exiting vessel 112. Additional configurations in which one or more oxygenated organic compounds (if formed) may be recovered from supported bio-catalyst 110 or vessel 112 are described in further detail below. Optionally, if sufficient methane conversion has not occurred, recycling of at least a portion of methane-depleted gas stream 120 exiting vessel 112 may occur via recycle line 130, thereby allowing longer residence times and a higher extent of methane conversion to be realized.

Monitoring locations 140 for assaying methane, carbon dioxide, carbon monoxide, or any combination thereof may be present to analyze methane-containing gas stream 102 before entering vessel 112, and/or methane-depleted gas stream 120 prior to release or recirculation via recycle line 130. One or multiple gas sensors capable of detecting methane, carbon dioxide, hydrogen sulfide, ammonia, and/or carbon monoxide may be present at monitoring locations 140. The same types of sensors need not necessarily be present at each of monitoring locations 140. Moreover, depending on application-specific needs, system 100 need not necessarily incorporate monitoring capabilities at each of monitoring locations 140. Additional monitoring locations 140 (not shown) may also be present.

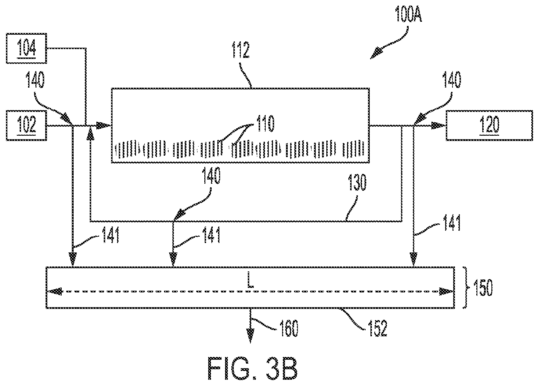

FIG. 3B shows a diagram of system 100A, in which one or more of monitoring locations 140 may be interrogated in a time-multiplexed manner with laser absorption spectroscopy system 150 *(e.g.,* a tunable diode laser absorption spectroscopy system), which may feature chamber 152 having path length L, and in which a laser and a detector (not shown) are located at opposing ends thereof. Lines 141 may divert a side stream of gas or gas mixture from monitoring locations 140 in a time-multiplexed manner (e.g., one at a time) and introduce the gas or gas mixture into laser absorption spectroscopy system 150 such that the gas or gas mixture fills chamber 152. It is to be appreciated that appropriate valves, pumps, control mechanisms therefor, and the like may be present in each of lines 141 and actuated at an appropriate time to provide the gas or gas mixture from one of monitoring locations 140 to chamber 152. After the gas or gas mixture has been analyzed in laser absorption spectroscopy system 150, chamber 152 may be evacuated via gas outlet 160, and gas or gas mixture from another of monitoring locations 140 may be provided to chamber 152 for analysis thereof. Additional monitoring locations 140 (not shown) may also be present.

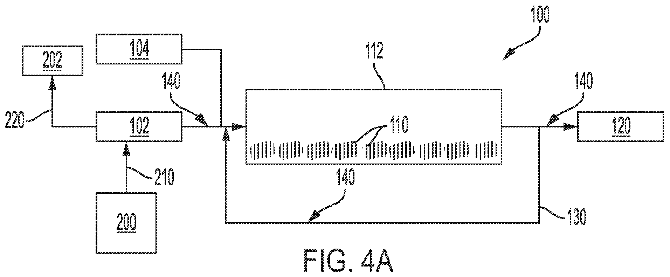

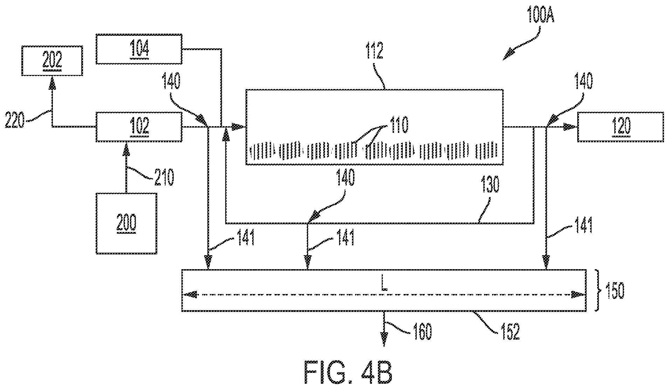

FIG. 4A is a diagram showing system 100 coupled to ventilation air of a mine to provide a source of methane-containing gas stream 102. As shown, ventilation air line 210 exits mine 200 and provides methane-containing gas stream 102 for further processing to remove methane therefrom according to the disclosure herein. A fan or pump (not shown) may facilitate transport of the ventilation air through ventilation air line 210. The entirety of the ventilation air may be provided to supported bio-catalyst 110, or some of the ventilation air may be released to the atmosphere without interacting with supported bio-catalyst 110 and removing methane therefrom. Some of the ventilation air may bypass supported bio-catalyst 110 if system 100 and ventilation air line 210 are slipstream-coupled to one another, for example. In another example, if the amount of ventilation air is unable to be effectively processed by the amount of supported bio-catalyst 110 present (even with recirculation occurring via recycle line 130), some of the ventilation air may be released to the atmosphere or diverted elsewhere for processing via diversion line 220. Unprocessed gas stream 202 may release a considerably lower volume of greenhouse methane than if the ventilation air in ventilation air line 210 was indiscriminately released without being processed according to the disclosure herein. In still another example, discussed further below, a portion of the ventilation air in ventilation air line 210 and diverted to unprocessed gas stream 202 via diversion line 220 may be provided to promote culturing of the methanotrophic microorganisms prior to forming a supported bio-catalyst of the present disclosure. FIG. 4B shows a diagram of corresponding system 100A coupled to ventilation air line 210 of mine 200.

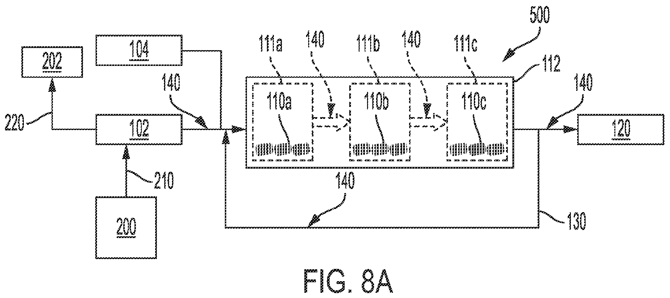

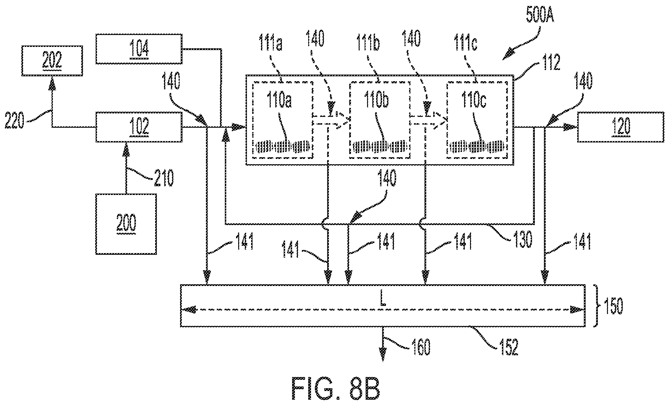

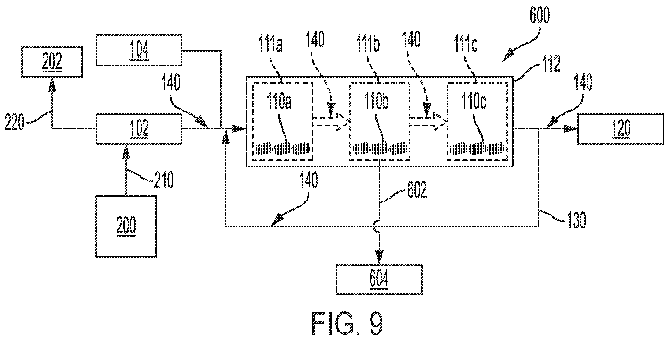

Any number of vessels 112 may be disposed in series and/or parallel in any embodiment of the disclosure herein, and the number of multiple vessels disclosed in the figures herein should be considered as illustrative and non-limiting. Alternately, a single vessel 112 may be present. Supported bio-catalyst 110 may also be implemented modularly within the systems of the present disclosure to provide a number of advantages. In the course of modular implementation, multiple vessels 112 may be disposed in series and/or parallel according to application-specific needs, as indicated above. Alternately, modular implementation may be accomplished in a single vessel 112. Additional details regarding modular implementation of supported bio-catalyst 102 are explained hereinafter in reference to FIGS. 8A, 8B, and 9.

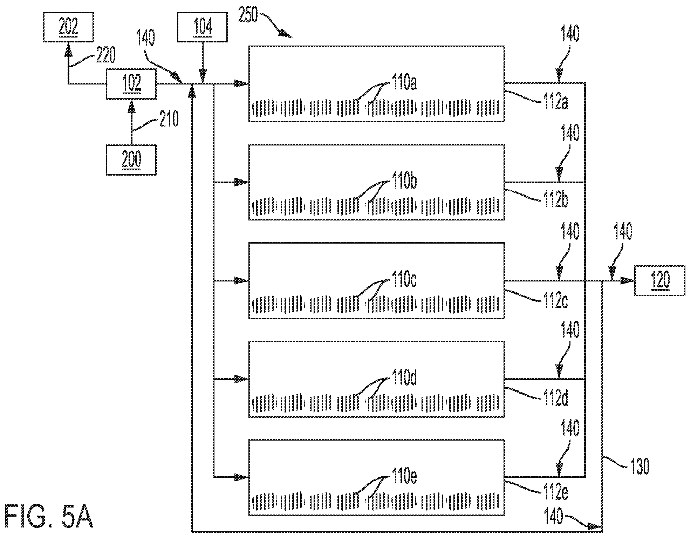

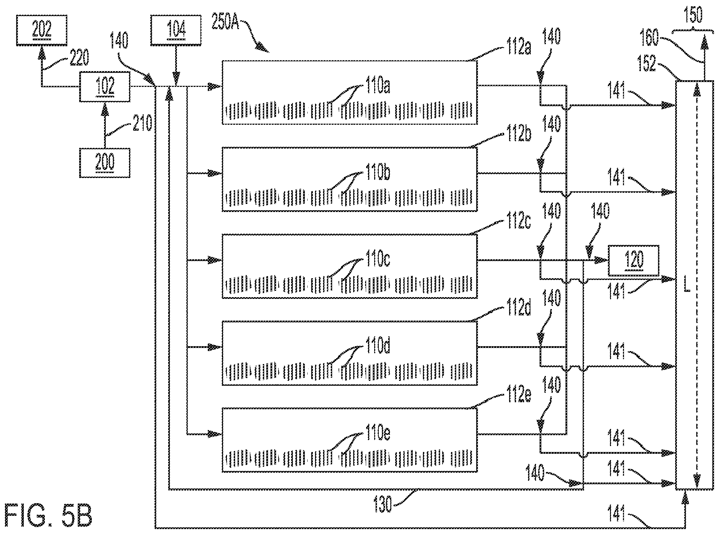

FIG. 5A is a diagram of a system containing multiple vessels arranged in parallel, each containing a supported bio-catalyst of the present disclosure. As shown, system 210 contains vessels 112a-112e disposed in parallel, each containing separate portions of supported bio-catalyst 110a-110e. System 250 is shown coupled to ventilation air line 210 of mine 200 to provide a source of methane-containing gas stream 102, in a similar manner to that shown in FIG. 4A. A combined gas stream exiting each of vessels 112a-112e, or a portion thereof, may be recycled upstream thereof via recycle line 130 in a manner similar to that described above. Although dedicated, individual recycle lines exiting each of vessels 112a-112e are not shown in FIG. 5A, it is to be appreciated that individual recycle lines may optionally be present, and a gas stream exiting one or more of vessels 112a-112e may be individually recycled upstream, for example, if the supported bio-catalyst in one or more of vessels 112a-112e but not others has become ineffective for converting methane or the methane abatement is otherwise not to the degree expected. Suitable monitoring locations 140 include a position upstream of vessels 112a-112e, a position downstream from each of vessels 112a-112e before recombination of the methane-depleted gas stream produced from each of vessels 112a-112e, a position downstream of a location where the methane-depleted gas streams from each of vessels 112a-112e are recombined, and within recycle line 130. It is to be further appreciated that although five vessels (112a-112e) are shown in parallel in FIG. 5A, more or fewer vessels and their associated supported bio-catalyst may be arranged in parallel, as dictated by application-specific requirements. FIG. 5B shows a diagram of corresponding system 250A, in which monitoring locations 140 are connected to laser absorption spectroscopy system 150 for time-multiplexed gas monitoring, in a manner similar to that described above for FIG. 3B.

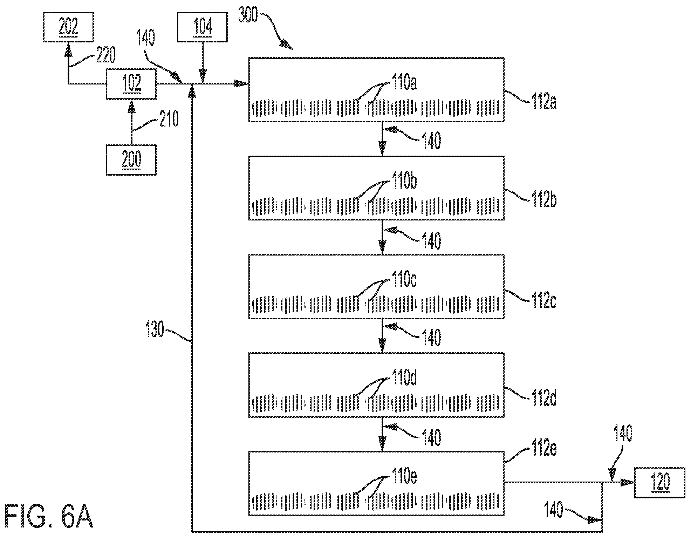

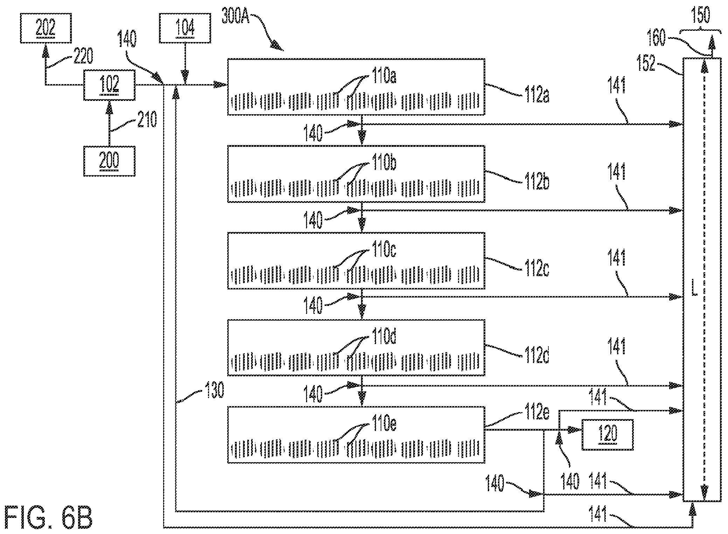

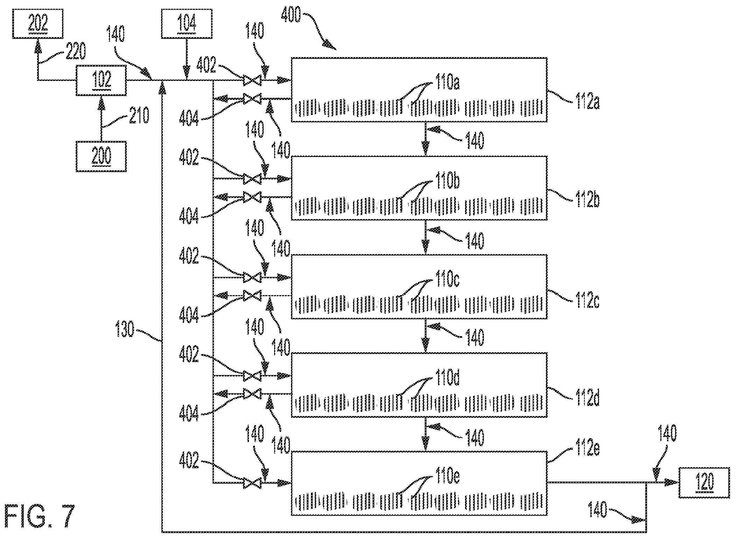

FIG. 6A is a diagram of a system containing multiple vessels arranged in series, each containing a supported bio-catalyst of the present disclosure. As shown, system 300 contains vessels 112a-112e disposed in series, each containing separate portions of supported bio-catalyst 110a-110e, wherein methane-containing gas stream 102 is provided first to vessel 112a and progresses sequentially to vessels 112b-112e before producing methane-depleted gas stream 120. The methane concentration may decrease upon sequential passage through each of vessels 112a-112e. System 300 is shown coupled to ventilation air line 210 of mine 200 to provide a source of methane-containing gas stream 102, in a similar manner to that provided in FIG. 4A. Suitable monitoring locations 140 may include a position upstream of vessel 112a, between each of vessels 112a-112d, downstream of vessel 112e, and within recycle line 130. Monitoring may allow one to determine if the supported bio-catalyst in any one or more of vessels 112a-112e has become ineffective for converting methane *(e.g.,* if the methane concentration does not decrease as expected after passing through a given example of vessels 112a-112e). Although not shown in system 300, it is to be appreciated that the methane-containing gas stream, or a portion thereof, may bypass one or more of vessels 112a-112e, as shown in further detail hereinafter in reference to FIG. 7 below. It is to be further appreciated that although five vessels (112a-112e) are shown in series in FIG. 6A, more or fewer vessels and their associated supported bio-catalyst may be arranged in series, as dictated by application-specific requirements. FIG. 6B shows a diagram of corresponding system 300A, in which monitoring locations 140 are connected to laser absorption spectroscopy system 150 for time-multiplexed gas monitoring, in a manner similar to that described above for FIG. 3B.

FIG. 7 is a diagram of a system containing multiple vessels arranged in both series and parallel, each vessel containing a supported bio-catalyst of the present disclosure. As shown, system 400 contains vessels 112a-112e disposed in series, each containing separate portions of supported bio-catalyst 110a-110e, wherein gas stream 102 is provided first to vessel 112a and progresses sequentially to vessels 112b-112e before producing methane-depleted gas stream 120. Vessels 112a-112e are also arranged in parallel with valves 402 and 404 being provided for each of vessels 112a-112d. Valves 402 and 404 may permit flow of methane-containing gas stream 102 to be interrupted (bypass) to any of vessels 112a-112d. System 400 is shown coupled to ventilation air line 210 of mine 200 to provide a source of methane-containing gas stream 102, in a similar manner to that provided in FIG. 4A. Although not shown in FIG. 7, methane-containing gas stream 102 may also bypass vessel 112e as well, with the effluent either exiting system 400 as methane-depleted gas stream 102 or being recirculated via recycle line 130. Interruption of methane-containing gas stream 102 to any of vessels 112a-112e may be desirable, for example, if satisfactory methane removal may be realized without the gas stream passing through each of vessels 112a-112e or if replacement of any of supported bio-catalysts 110a-110e is needed or required and/or maintenance on any of vessels 112a-112e needs to take place. For example, if supported bio-catalyst 110b needs to be replaced, gas flow may be temporarily interrupted to vessels 112a and 112b, while gas flow still continues through vessels 112c-112e, thereby allowing methane removal to continue uninterrupted, optionally at a lower flow rate. Suitable monitoring locations 140 may include a position upstream of vessel 112a, between each of vessels 112a-112d, downstream of vessel 112e, proximal to valves 402 and/or 404, and within recycle line 130. It is to be appreciated that although five vessels (112a-112e) are shown in FIG. 7, more or fewer vessels and their associated supported bio-catalyst may be present, as dictated by application-specific requirements. Moreover, one or more of monitoring locations 140 may be coupled to a laser absorption spectroscopy system (e.g., laser absorption spectroscopy system 150 in FIG. 3B) in a manner similar to that described above. In the interest of brevity, this additional configuration is not shown herein.

The supported bio-catalyst may also be implemented modularly within a single vessel (reactor) as well or within multiple vessels, with individual bio-catalyst modules being located in series and/or parallel. FIG. 8A is a diagram of a system containing multiple supported bio-catalyst modules housed in a single vessel. As shown, system 500 includes modules 111a-111c, containing supported bio-catalyst 110a-110c, respectively, housed within vessel 112. System 500 is otherwise similar to system 100, shown in FIGS. 3A and 4A, and may be better understood by reference thereto. In operation of system 500, methane-containing gas stream 102 enters vessel 112 and sequentially proceeds through modules 111a-111c. The gas flow between modules 111a, 112b and 111c is shown with block arrows in FIG. 8A. Advantageously, modules 111a-111c may be removed from vessel 112 individually, should replacement or further processing of one of supported bio-catalysts 110a-110c be needed. For example, in the event that oxygenated organic compounds are produced and remain sequestered within any of supported bio-catalysts 110a-110c, modules 111a-111c may be replaced individually or sequentially, and the oxygenated organic compounds may be recovered therefrom. Since oxygenated organic compounds may be conducted without removing the entirety of the supported bio-catalyst, processing of methane-containing gas stream 102 may continue substantially uninterrupted while any of modules 111a-111c are being replaced and/or oxygenated organic compounds are being harvested. Monitoring locations 140 are similar to those shown in FIGS. 3A and 4A, with the addition of further monitoring locations 140 between modules 111a and 111b and between modules 111b and 111c, which may provide guidance as to the performance of individual modules 111a-111c. FIG. 8B shows a diagram of corresponding system 500A, in which monitoring locations 140 are connected to laser absorption spectroscopy system 150 for time-multiplexed gas monitoring, in a manner similar to that described above for FIG. 3B.

Oxygenated organic compounds may be recovered from the supported bio-catalyst in any of the system configurations disclosed herein, either by removing at least a portion of the supported bio-catalyst from the vessel in which it is housed or a catalyst module containing the supported bio-catalyst. Recovery of the oxygenated organic compounds may take place, for example, by lysing the methanotrophic microorganisms and extracting the oxygenated organic compounds from the lysate with a suitable solvent.

In some instances, oxygenated organic compounds, if produced, may not be retained by the supported bio-catalyst, in which case the oxygenated organic compounds may collect at the bottom of the vessel or catalyst module in which the supported bio-catalyst is housed. In the event oxygenated organic compounds are not retained by the supported bio-catalyst, the oxygenated organic compounds may simply be drained from the bottom of the vessel or catalyst module and collected.

FIG. 9 is a diagram of a system in which oxygenated organic compounds may be drained from a vessel containing a supported bio-catalyst. As shown, system 600 features drain line 602 extending from vessel 112 to collect oxygenated organic compounds in collection basin 604. System 600 is otherwise similar to system 500 in FIG. 8 and may be better understood by reference thereto. System 600 may similarly be coupled to a laser absorption spectroscopy system (e.g., laser absorption spectroscopy system 150) in a similar manner to that described above in reference to FIG. 3B. Drain line 602 and collection basin 604 may be incorporated within any of the other system configurations disclosed herein as well, including those in which the supported bio-catalyst is or is not modularly incorporated. It is to be further appreciated that drain line 602 may be positioned upon vessel 112 in a location to facilitate drainage therefrom and/or vessel 112 may be shaped to facilitate drainage. For example, vessel 112 may be conical and/or have a sloped bottom surface to promote drainage of oxygenated organic compounds and other liquids therefrom.

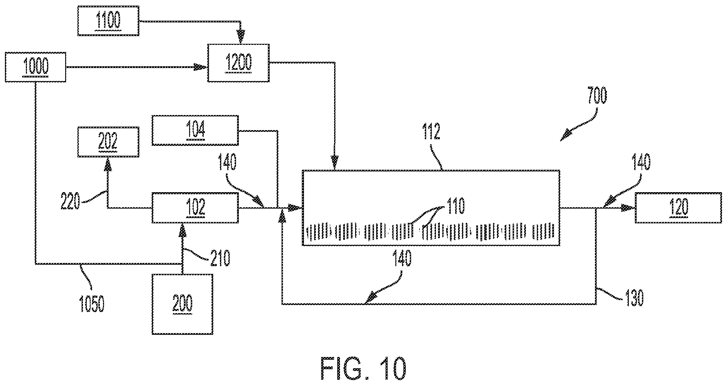

Advantageously, the ready source of methane at a job site may allow methanotrophic microorganisms, such as methanotrophic bacteria, to be cultured and incorporated upon a porous support material onsite prior to being incorporated in a system for removal of methane from a methane-containing gas stream. FIG. 10 is a diagram showing how onsite culturing and incorporation of methanotrophic microorganisms upon a porous support material may take place. Other than incorporating culturing and immobilization of methanotrophic microorganisms, system 700 in FIG. 10 is similar to system 100 in FIGS. 3A and 4A and may be better understood by reference thereto. As shown, system 700 withdraws a portion of the mine ventilation air from ventilation air line 210 via line 1050 and provides the methane-containing gas to bioreactor 1000, in which methanotrophic microorganisms may be cultured. Mine ventilation air supplied by line 1050 may be diluted ventilation air below the methane combustion limit, or the mine ventilation air supplied to the methanotrophic microorganisms may be drawn from an area of the mine having a higher methane concentration (or the ventilation air may be undiluted), thereby supplying the methanotrophic microorganisms with a richer methane nutrient source to facilitate culturing. The methanotrophic microorganisms may then be combined with a porous support material from source 1100 or a precursor thereto to form a supported bio-catalyst, which may then be introduced to vessel 112 from holding location 1200 at a desired time. The supported bio-catalyst from holding location 1200 may be introduced to vessel 112 modularly or as a free powder, a cast porous slab, and/or a structured lattice. It is to be appreciated that culturing and methanotrophic microorganism immobilization may be incorporated in any of the other system configurations disclosed herein as well. Moreover, system 700 may similarly be coupled to a laser absorption spectroscopy system *(e.g.,* laser absorption spectroscopy system 150) in a similar manner to that described above in reference to FIG. 3B.

In non-limiting examples, monitoring data received from any of the monitoring locations described above may be utilized reactively to determine if a given portion of the supported bio-catalyst is functioning as intended and/or needs to be replaced. Likewise, monitoring data received from one or more monitoring locations may be utilized reactively to determine if discharge standards specified in local environmental regulations are being met. If it is determined that discharge standards are not being met and/or any portion of the supported bio-catalyst is not functioning as intended, the supported bio-catalyst may be replaced or corrective process action may be taken (*e.g.,* increasing oxygen content supplied with the methane-containing gas stream, decreasing gas flow rates, adjusting the temperature in a vessel housing the supported bio-catalyst, changing recirculation rates, and the like).

Monitoring data received from any of the monitoring locations may similarly be utilized proactively to aid in determining the "health" of the supported bio-catalyst or any portion thereof. For example, multivariate linear regression may be utilized to estimate the probable remaining lifetime of the supported bio-catalyst or any portion thereof. In non-limiting examples, monitoring data (or any other available data from a system of the present disclosure, such as gas flow rates, pressures, temperatures, or the like) may be analyzed to determine the current state of the supported bio-catalyst and how much longer it likely may be effectively used. For example, factors that may be taken into account for determining the current state and anticipated remaining lifetime of the supported bio-catalyst include, but are not limited to, the methane-containing gas stream flow rate, the methane content of the methane-containing gas stream, the presence of poisons in the methane-containing gas stream (e.g., carbon monoxide, hydrogen sulfide, ammonia, higher alkanes, or the like), and how long and at what concentration the poisons have been exposed to the supported bio-catalyst. Such analyses may be correlated to the carbon dioxide or oxygenated organic compounds produced by the supported bio-catalyst or any portion thereof. Such understanding and correlation may be based upon historical performance of the methanotrophic microorganisms under comparable and known laboratory or culturing conditions. When there are decreased carbon dioxide or oxygenated organic compound emissions, the data may be analyzed to determine if the decreased emissions are systemic and indicative of decreasing bio-catalyst performance, or transient as a result of momentarily increased poison concentrations in the methane-containing gas stream or some other type of process upset. Similarly, increased carbon dioxide production beyond that expected based upon the amount of methane present may be correlated with increased metabolic activity of the supported bio-catalyst (particularly when occurring in conjunction with a coincident increase in methane input) or diagnostic of the supported bio-catalyst undergoing degradation, such as when bacterial cell degradation occurs to produce carbon dioxide, and there is not a concurrent improvement in methane abatement.

To afford proactive or reactive processing of system data, data collected from the monitoring locations may be conveyed in a wired or wireless manner to a computer or computer system configured to analyze the data and make determinations, such as those described above. Any suitable electronic communication protocol may be used for wireless communication of data, such as near field communication (NFC), radio frequency identification (RFID), BLUETOOTH^{®} or BLUETOOTH^{®} Low Energy protocols, WiFi, or the like. The computer or computer system may comprise a processor; a memory coupled to the processor; and instructions provided to the memory, such that the instructions are executable by the processor to make one or more of the determinations specified above. That is, the instructions may perform multivariate linear regression to make one or more of the determinations specified above for assessing performance of the supported bio-catalyst. The computer or computer system may be further configured to regulate a system or process based on the one or more determinations. For example, the computer or computer system may autonomously adjust one or more parameters such as oxygen content of the methane-containing gas stream, gas flow rates, temperature or the like in response to the current state or anticipated remaining lifetime of the supported bio-catalyst. In another example, the computer or computer system may adjust one or more of such parameters in response to a change in composition of the methane-containing gas stream being provided to the supported bio-catalyst, such as due to an unexpected increase in the concentration of a poison, such as carbon monoxide, ammonia, and/or hydrogen sulfide. Alternately, the computer or computer system may provide recommended alterations to one or more of such parameters, such that an operator may manually make the alterations or conduct other interventions at their discretion.

Accordingly, the systems and methods of the present disclosure may incorporate a non-transitory computer readable medium containing instructions that, when implemented, cause one or more processors to carry out one or more determinations, such as those specified above. "Computer-readable medium" or "non-transitory, computer-readable medium," as used herein, refers to any non-transitory storage and/or transmission medium that participates in providing instructions to a processor for execution. Such a medium may include, but is not limited to, non-volatile media and volatile media. Non-volatile media includes, for example, NVRAM, or magnetic or optical disks. Volatile media includes dynamic memory, such as main memory. Common forms of computer-readable media include, for example, a floppy disk, a flexible disk, a hard disk, an array of hard disks, a magnetic tape, or any other magnetic medium, magneto-optical medium, a CD-ROM, a holographic medium, any other optical medium, a RAM, a PROM, an EPROM, a FLASH-EPROM, a solid state medium like a memory card, any other memory chip or cartridge, or any other tangible medium from which a computer can read data or instructions. When the computer-readable media is configured as a database, it is to be understood that the database may be any type of database, such as relational, hierarchical, object-oriented, and/or the like. Accordingly, exemplary embodiments of the systems and methods may include a tangible storage medium, a tangible distribution medium, equivalents thereof, and successor media thereof, in which software implementations for carrying out one or more determinations based upon multivariate linear regression may be accomplished.

Any suitable processor-based device may be utilized for implementing all or a portion of one or more of the determinations specified above, including without limitation, personal computers, networks of personal computers, laptop computers, computer workstations, mobile devices, multiprocessor servers or workstations with (or without) shared memory, high performance computers, cloud-based computers, and the like. Application specific integrated circuits (ASICs) or very large scale integrated (VLSI) circuits may also be utilized in this regard.

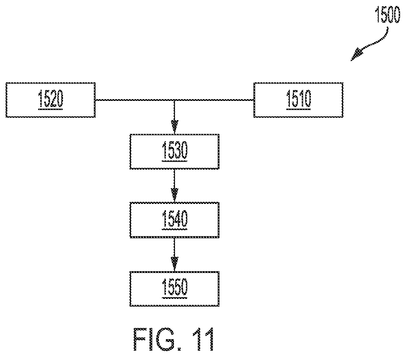

FIGS. 11-13 are process diagrams showing how methanotrophic microorganisms may be immobilized upon a porous support material. The methanotrophic microorganisms, such as methanotrophic bacteria, may be cultured at a job site (see FIG. 10) with the supported bio-catalysts may be prepared onsite, or the supported bio-catalysts may be prepared offsite *(e.g.,* in a laboratory or processing facility) and transported to a job site. If transported to a job site (or stored in bulk), the supported bio-catalysts may be transported (or stored) while infiltrated with a liquid, such as methanol, liquid alkanes, or other small carbon compounds, to provide a nutrient source for the methanotrophic microorganisms. Alternately, the supported bio-catalyst may be transported to a job site (or stored) under a methane atmosphere.

FIG. 11 is a diagram of process 1500, in which methanotrophic microorganisms 1510 (cultured at a job site or elsewhere) may be combined with precursor blend 1520 comprising particles and a polymer binder, either in the form of pre-synthesized polymers or polymerizable monomers. The polymer binder, particles and clusters of methanotrophic microorganisms may be polymerized or solvated together in operation 1530, followed by drying and solidification 1540, to afford supported bio-catalyst 1550. Operation 1530 and drying and solidification 1540 may include adjusting various process variables such as temperature, pressure, pH, and the like to promote formation of a specified morphology within the porous support structure of supported bio-catalyst 1550.

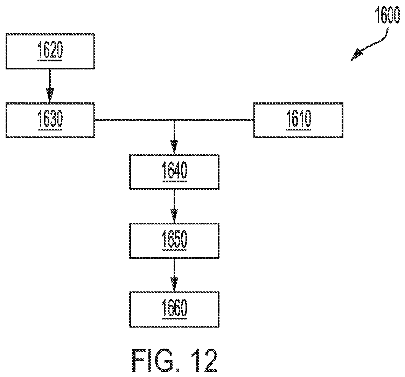

FIG. 12 is a diagram of process 1600, in which methanotrophic microorganisms (cultured at a job site or elsewhere) may be combined directly with a porous support material previously prepared from polymerizable monomers and particles. As shown, precursor blend 1620 comprising polymerizable monomers and particles undergoes polymerization together to afford porous support material 1630. Methanotrophic microorganisms 1610 and porous support material 1630 are combined in operation 1640. Following infiltration 1640 of clusters of methanotrophic microorganisms into porous support material 1630 and drying 1650, supported bio-catalyst 1660 is obtained.

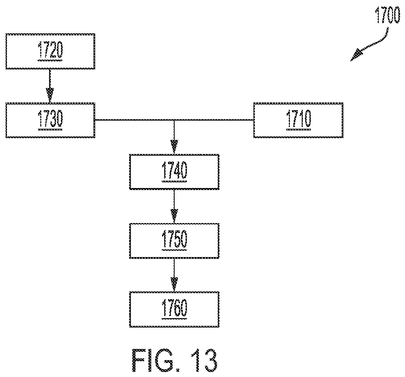

FIG. 13 is a diagram of process 1700, in which methanotrophic microorganisms (cultured at a job site or elsewhere) may be combined directly with a porous support material previously prepared from a mixture of polymerizable monomers and solvent. As shown, monomer/solvent blend 1720 is formed into a desired shape and undergoes polymerization together, followed by solvent removal, to afford porous support material 1730. Alternately, monomer/solvent blend 1720 may instead be a polymer/solvent blend, in which case following shaping, no additional polymerization takes place prior to solvent removal to form porous support material 1730. Methanotrophic microorganisms 1710 and porous support material 1730 are combined in operation 1740. Following infiltration of clusters of methanotrophic microorganisms into porous support material 1730 and drying 1750, supported bio-catalyst 1760 is obtained.

Embodiments disclosed herein include:
A. Supported bio-catalysts. The supported bio-catalysts comprise: a porous support material; and a plurality of microorganism clusters immobilized within pores of the porous support material, one or more microorganisms within the microorganism clusters being effective to convert methane into one or more oxidized carbon compounds in substantial absence of a liquid phase.
B. Methods for processing a methane-containing gas stream. The methods comprise: interacting a methane-containing gas stream with a supported bio-catalyst in substantial absence of a liquid phase, the supported bio-catalyst comprising: a porous support material; and a plurality of microorganism clusters immobilized within pores of the porous support material, one or more microorganisms within the microorganism clusters being effective to convert methane into one or more oxidized carbon compounds; and obtaining a methane-depleted gas stream downstream from the supported bio-catalyst.
C. Systems for processing a methane-containing gas stream. The systems comprise: a gas inlet fluidly coupled to one or more vessels, the gas inlet providing a methane-containing gas stream to the one or more vessels and the one or more vessels housing a supported bio-catalyst comprising: a porous support material; and a plurality of microorganism clusters immobilized within pores of the porous support material, microorganisms within the microorganism clusters being effective to convert methane into one or more oxidized carbon compounds in substantial absence of a liquid phase; and a gas outlet fluidly coupled to at least a portion of the one or more vessels and configured to remove a methane-depleted gas stream therefrom.
D. A bio-catalyst module comprising the supported bio-catalyst of A.

Each of embodiments A-D may have one or more of the following additional elements in any combination:
Element 1: wherein the microorganism clusters comprise at least one type of methanotrophic bacteria.
Element 2: wherein the at least one type of methanotrophic bacteria comprises at least *Methylotuvimicrobium alcaliphilum sp. 20Z.*
Element 3: wherein the at least one type of methanotrophic bacteria is effective to convert methane into carbon dioxide.
Element 4: wherein the at least one type of methanotrophic bacteria is effective to convert methane into an oxygenated organic compound.
Element 5: wherein the at least one type of methanotrophic bacteria is genetically modified.
Element 6: wherein the supported bio-catalyst further comprises: a nutrient source for the methanotrophic bacteria present within pores of the porous support material.
Element 6A: wherein the method further comprises: providing a nutrient source to the at least one type of methanotrophic bacteria prior to interacting the methane-containing gas stream with the supported bio-catalyst. Optionally, the nutrient source is a liquid.
Element 7: wherein the nutrient source comprises methanol or a liquid alkane.
Element 8: wherein the one or more oxidized carbon compounds comprise one or more oxygenated organic compounds.
Element 9: wherein the method further comprises: obtaining at least a portion of the one or more oxygenated organic compounds from the supported bio-catalyst or a vessel housing the supported bio-catalyst.
Element 10: wherein the methane-containing gas stream is obtained from mining ventilation.
Element 11: wherein the method further comprises introducing air or oxygen into the methane-containing gas stream.
Element 12: wherein the method further comprises recirculating at least a portion of the methane-depleted gas stream to the supported bio-catalyst.
Element 13: wherein the supported bio-catalyst is housed in one or more vessels and the methane-containing gas stream is introduced to the one or more vessels.
Element 14: wherein the supported bio-catalyst is incorporated modularly in at least a portion of the one or more vessels.
Element 15: wherein a methane content of the methane-containing gas stream is about 20 vol. % or less.
Element 16: wherein a methane content of the methane-containing gas stream is about 5 vol. % or less.
Element 17: wherein the one or more vessels comprise multiple vessels.
Element 18: wherein the multiple vessels are disposed in series, parallel, or any combination thereof.
Element 19: wherein the system further comprises an oxygen supply configured to provide air or oxygen to the methane-containing gas stream and/or to at least a portion of the one or more vessels.
Element 20: wherein the system further comprises a recycle line configured to recirculate at least a portion of the methane-depleted gas stream to at least a portion of the one or more vessels.
Element 21: wherein the system further comprises a drain line configured to remove an oxygenated organic compound from at least a portion of the one or more vessels.
Element 22: wherein the methane-containing gas stream is received from mining ventilation.
Element 23: wherein the methane-containing gas stream is further fluidly coupled to a culture medium for growing the microorganism clusters.
Element 24: wherein the method further comprises determining a concentration of one or more gases in the methane-containing gas stream, the methane-depleted gas stream, or any combination thereof at one or more monitoring locations.
Element 25: wherein the one or more monitoring locations are multiple monitoring locations.
Element 25A: wherein the concentration of the one or more gases is determined at multiple monitoring locations using a gas sensor present at each monitoring location, or at multiple monitoring locations using a laser absorption spectroscopy system.
Element 26: wherein the concentration of the one or more gases are determined using a laser absorption spectroscopy system.
Element 27: wherein the one or more gases are methane, carbon dioxide, carbon monoxide, or any combination thereof.
Element 28: wherein at least a methane concentration is measured at each of the one or more monitoring locations.
Element 29: wherein the method further comprises determining a current state or an anticipated remaining lifetime of the supported bio-catalyst based upon the concentration of the one or more gases determined at the one or more monitoring locations.
Element 30: wherein the system further comprises one or more monitoring locations for determining a concentration of one or more gases in the methane-containing gas stream, the methane-depleted gas stream, or any combination thereof; wherein a gas sensor is present at each of the one or more monitoring locations, the one or more monitoring locations are connected to a laser absorption spectroscopy system, or any combination thereof.
Element 31: wherein the system is further configured to determine a current state or an anticipated remaining lifetime of the supported bio-catalyst based upon the concentration of the one or more gases determined at the one or more monitoring locations.

By way of non-limiting example, exemplary combinations applicable to A-D include, but are not limited to: one or more of 1-5, and 6 or 6A; one or more of 1-5, 6 or 6A, and 7; one or more of 1-5, and 8; one or more of 1-5, 8 and 9; one or more of 1-5, and 10; one or more of 1-5, and 11 or 19; one or more of 1-5, 10 and 11; one or more of 1-5, and 12; one or more of 1-5, and 13; one or more of 1-5, and 14; one or more of 1-5, and 15 or 16; one or more of 1-5, and 17; one or more of 1-5, and 18; one or more of 1-5, and 19; one or more of 1-5, and 20; one or more of 1-5, and 21; one or more of 1-5, and 22; one or more of 1-5, and 23; 8 and 9; 8 and 10; 8 and 11; 8, 10 and 11; 8 and 12; 8 and 13; 8 and 14; 8, and 15 or 16; 8 and 17; 8, 17 and 18; 10 and 11; 10 and 12; 10 and 13; 10 and 14; 10, and 15 or 16; 10 and 17; 10, 17 and 18; 11 and 12; 11 and 13; 11 and 14; 11, and 15 or 16; 12 and 13; 12 and 14; 12, and 15 or 16; 17 and 18; 17 and 19; 17 and 20; 17 and 21; 17 and 22; 17 and 23; 19 and 20; 19 and 21; 19 and 22; 19 and 23; 20 and 21; 20 and 22; 20 and 23; 21 and 22; 21 and 23; and 22 and 23. Any of the foregoing may be present in further combination with one or more of 24-31. Additional exemplary combinations applicable to C and D include, but are not limited to, 24, and 25 or 25A; 24 and 26; 24 and 27; 24 and 28; 24 and 29; 25 or 25A, and 26; 25 or 25A, and 27; 25 or 25A, and 28; 25 or 25A, and 29; 25 or 25A, and 30; 25 or 25A, and 31; 26 and 27; 26 and 28; 26 and 29; 26 and 30; 26 and 31; 27 and 29; 28 and 29; 28 and 30; 28 and 31; and 30 and 31.

The present disclosure is also encompassed by the following non-limiting clauses:
Clause 1: A supported bio-catalyst, comprising:
   a porous support material; and
   a plurality of microorganism clusters immobilized within pores of the porous support material, one or more microorganisms within the microorganism clusters being effective to convert methane into one or more oxidized carbon compounds in substantial absence of a liquid phase.
Clause 2: The supported bio-catalyst of Clause 1, wherein the microorganism clusters comprise at least one type of methanotrophic bacteria.
Clause 3: The supported bio-catalyst of Clause 2, wherein the at least one type of methanotrophic bacteria comprises at least *Methylotuvimicrobium alcaliphilum sp. 20Z.*
Clause 4: The supported bio-catalyst of Clause 2 or Clause 3, wherein the at least one type of methanotrophic bacteria is effective to convert methane into carbon dioxide or an oxygenated organic compound.
Clause 5: The supported bio-catalyst of any of Clauses 2 to 4, further comprising:
   a nutrient source for the methanotrophic bacteria present within pores of the porous support material.
Clause 6: A bio-catalyst module comprising the supported bio-catalyst of any of Clauses 1 to 4.
Clause 7: A method comprising:
   interacting a methane-containing gas stream with a supported bio-catalyst in substantial absence of a liquid phase, the supported bio-catalyst comprising:
      a porous support material; and
      a plurality of microorganism clusters immobilized within pores of the porous support material, one or more microorganisms within the microorganism clusters being effective to convert methane into one or more oxidized carbon compounds; and
   obtaining a methane-depleted gas stream downstream from the supported bio-catalyst.
Clause 8: The method of Clause 7, wherein the microorganism clusters comprise at least one type of methanotrophic bacteria.
Clause 9: The method of Clause 8, wherein the at least one type of methanotrophic bacteria comprises at least *Methylotuvimicrobium alcaliphilum sp. 20Z.*
Clause 10: The method of Clause 8 or Clause 9, wherein the at least one type of methanotrophic bacteria is effective to convert methane into carbon dioxide or an oxygenated organic compound.
Clause 11: The method of any of Clause 8 to 10, further comprising:
   providing a nutrient source to the at least one type of methanotrophic bacteria within the supported bio-catalyst prior to interacting the methane-containing gas stream with the supported bio-catalyst.
Clause 12: The method of any of Clauses 7 to 11, wherein the one or more oxidized carbon compounds comprise one or more oxygenated organic compounds.
Clause 13: The method of Clause 12, further comprising:
   obtaining at least a portion of the one or more oxygenated organic compounds from the supported bio-catalyst or a vessel housing the supported bio-catalyst.
Clause 14: The method of any of Clauses 7 to 13, wherein the methane-containing gas stream is obtained from mining ventilation.
Clause 15: The method of any of Clauses 7 to 14, further comprising:
   introducing air or oxygen into the methane-containing gas stream.
Clause 16: The method of any of Clauses 7 to 15, further comprising:
   recirculating at least a portion of the methane-depleted gas stream to the supported bio-catalyst.
Clause 17: The method of any of Clauses 7 to 16, wherein a methane content of the methane-containing gas stream is about 20 vol. % or less.
Clause 18: The method of any of Clauses 7 to 17, further comprising:
   determining a concentration of one or more gases in the methane-containing gas stream, the methane-depleted gas stream, or any combination thereof at one or more monitoring locations. Clause 19: The method of Clause 18, wherein the concentration of the one or more gases is determined at multiple monitoring locations using a gas sensor present at each monitoring location, or at multiple monitoring locations using a laser absorption spectroscopy system.
Clause 20: The method of Clause 18 or Clause 19, further comprising:
   determining a current state or an anticipated remaining lifetime of the supported bio-catalyst based upon the concentration of the one or more gases determined at the one or more monitoring locations.
Clause 21: A system comprising:
   a gas inlet fluidly coupled to one or more vessels, the gas inlet providing a methane-containing gas stream to the one or more vessels and the one or more vessels housing a supported bio-catalyst comprising:
      a porous support material; and
      a plurality of microorganism clusters immobilized within pores of the porous support material, one or more microorganisms within the microorganism clusters being effective to convert methane into one or more oxidized carbon compounds in substantial absence of a liquid phase; and
   a gas outlet fluidly coupled to at least a portion of the one or more vessels and configured to remove a methane-depleted gas stream therefrom.
Clause 22: The system of Clause 21, wherein the microorganism clusters comprise at least one type of methanotrophic bacteria.
Clause 23: The system of Clause 22, wherein the at least one type of methanotrophic bacteria comprises at least *Methylotuvimicrobium alcaliphilum sp. 20Z.*
Clause 24: The system of Clause 22 or Clause 23, wherein the at least one type of methanotrophic bacteria is effective to convert methane into carbon dioxide or an oxygenated organic compound.
Clause 25: The system of any of Clauses 21 to 24, wherein the one or more vessels comprise multiple vessels disposed in series, parallel, or any combination thereof.
Clause 26: The system of any of Clauses 21 to 25, further comprising one or more of:
   a) an oxygen supply configured to provide air or oxygen to the methane-containing gas stream and/or to at least a portion of the one or more vessels,
   b) a recycle line configured to recirculate at least a portion of the methane-depleted gas stream to at least a portion of the one or more vessels, or
   c) a drain line configured to remove an oxygenated organic compound from at least a portion of the one or more vessels.
Clause 27: The system of any of Clauses 21 to 26, wherein the supported bio-catalyst is incorporated modularly in at least a portion of the one or more vessels.
Clause 28: The system of any of Clauses 21 to 27, wherein the methane-containing gas stream is further fluidly coupled to a culture medium for growing the microorganism clusters.
Clause 29: The system of any of Clauses 21 to 28, further comprising:
   one or more monitoring locations for determining a concentration of one or more gases in the methane-containing gas stream, the methane-depleted gas stream, or any combination thereof;
   wherein a gas sensor is present at each of the one or more monitoring locations, the one or more monitoring locations are connected to a laser absorption spectroscopy system, or any combination thereof.

All documents described herein are incorporated by reference herein for purposes of all jurisdictions where such practice is allowed, including any priority documents and/or testing procedures to the extent they are not inconsistent with this text. As is apparent from the foregoing general description and the specific embodiments, while forms of the disclosure have been illustrated and described, various modifications can be made without departing from the spirit and scope of the disclosure. Accordingly, it is not intended that the disclosure be limited thereby. For example, the compositions described herein may be free of any component, or composition not expressly recited or disclosed herein. Any method may lack any step not recited or disclosed herein. Likewise, the term "comprising" is considered synonymous with the term "including." Whenever a method, composition, element or group of elements is preceded with the transitional phrase "comprising," it is understood that we also contemplate the same composition or group of elements with transitional phrases "consisting essentially of," "consisting of," "selected from the group of consisting of," or "is" preceding the recitation of the composition, element, or elements and vice versa.

Unless otherwise indicated, all numbers expressing quantities of ingredients, properties such as molecular weight, reaction conditions, and so forth used in the present specification and associated claims are to be understood as being modified in all instances by the term "about." Accordingly, unless indicated to the contrary, the numerical parameters set forth in the following specification and attached claims are approximations that may vary depending upon the desired properties sought to be obtained by the embodiments of the present invention. At the very least, and not as an attempt to limit the application of the doctrine of equivalents to the scope of the claim, each numerical parameter should at least be construed in light of the number of reported significant digits and by applying ordinary rounding techniques.

Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the element that it introduces.

One or more illustrative embodiments are presented herein. Not all features of a physical implementation are described or shown in this application for the sake of clarity. It is understood that in the development of a physical embodiment of the present disclosure, numerous implementation-specific decisions must be made to achieve the developer's goals, such as compliance with system-related, business-related, government-related and other constraints, which vary by implementation and from time to time. While a developer's efforts might be time-consuming, such efforts would be, nevertheless, a routine undertaking for one of ordinary skill in the art and having benefit of this disclosure.

Therefore, the present disclosure is well adapted to attain the ends and advantages mentioned as well as those that are inherent therein. The particular embodiments disclosed above are illustrative only, as the present disclosure may be modified and practiced in different but equivalent manners apparent to one having ordinary skill in the art and having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope and spirit of the present disclosure. The embodiments illustratively disclosed herein suitably may be practiced in the absence of any element that is not specifically disclosed herein and/or any optional element disclosed herein.

## Claims

1. A supported bio-catalyst, comprising:
a porous support material; and
a plurality of microorganism clusters immobilized within pores of the porous support material, one or more microorganisms within the microorganism clusters being effective to convert methane into one or more oxidized carbon compounds in substantial absence of a liquid phase.

2. The supported bio-catalyst of claim 1, wherein the microorganism clusters comprise at least one type of methanotrophic bacteria, optionally wherein the at least one type of methanotrophic bacteria comprises at least *Methylotuvimicrobium alcaliphilum sp. 20Z,* optionally wherein the at least one type of methanotrophic bacteria is effective to convert methane into carbon dioxide or an oxygenated organic compound.

3. The supported bio-catalyst of claim 2, further comprising:
a nutrient source for the methanotrophic bacteria present within pores of the porous support material.

4. A bio-catalyst module comprising the supported bio-catalyst as defined in any of claims 1 to 3.

5. A method comprising:
providing a methane-containing gas stream, optionally a methane-containing gas stream obtained from mining ventilation;
optionally introducing air or oxygen into the methane-containing gas stream;
interacting the methane-containing gas stream with the supported bio-catalyst of any of claims 1 to 3 in the substantial absence of a liquid phase;
obtaining a methane-depleted gas stream downstream from the supported bio-catalyst; and
optionally recirculating at least a portion of the methane-depleted gas stream to the supported bio-catalyst.

6. The method of claim 5, further comprising:
providing a nutrient source to the at least one type of methanotrophic bacteria within the supported bio-catalyst prior to interacting the methane-containing gas stream with the supported bio-catalyst.

7. The method of claim 5 or claim 6, wherein the one or more oxidized carbon compounds comprise one or more oxygenated organic compounds and wherein the method optionally further comprises obtaining at least a portion of the one or more oxygenated organic compounds from the supported bio-catalyst or a vessel housing the supported bio-catalyst.

8. The method of any of claims 5 to 7, wherein a methane content of the methane-containing gas stream is about 20 vol. % or less.

9. The method of any of claims 5 to 8, further comprising:
determining a concentration of one or more gases in the methane-containing gas stream, the methane-depleted gas stream, or any combination thereof at one or more monitoring locations, optionally wherein the concentration of the one or more gases is determined at multiple monitoring locations using a gas sensor present at each monitoring location, or at multiple monitoring locations using a laser absorption spectroscopy system.

10. The method of claim 9, further comprising:
determining a current state or an anticipated remaining lifetime of the supported bio-catalyst based upon the concentration of the one or more gases determined at the one or more monitoring locations.

11. A system comprising:
a gas inlet fluidly coupled to one or more vessels, the gas inlet providing a methane-containing gas stream to the one or more vessels and the one or more vessels housing a supported bio-catalyst as defined in any of claims 1 to 3, optionally wherein the supported bio-catalyst is incorporated modularly in at least a portion of the one or more vessels; and
a gas outlet fluidly coupled to at least a portion of the one or more vessels and configured to remove a methane-depleted gas stream therefrom.

12. The system of claim 11, wherein the one or more vessels comprise multiple vessels disposed in series, parallel, or any combination thereof.

13. The system of claim 11 or claim 12, further comprising one or more of:
a) an oxygen supply configured to provide air or oxygen to the methane-containing gas stream and/or to at least a portion of the one or more vessels,
b) a recycle line configured to recirculate at least a portion of the methane-depleted gas stream to at least a portion of the one or more vessels, or
c) a drain line configured to remove an oxygenated organic compound from at least a portion of the one or more vessels.

14. The system of any of claims 11 to 13, wherein the methane-containing gas stream is further fluidly coupled to a culture medium for growing the microorganism clusters.

15. The system of any of claims 11 to 14, further comprising:
one or more monitoring locations for determining a concentration of one or more gases in the methane-containing gas stream, the methane-depleted gas stream, or any combination thereof;
wherein a gas sensor is present at each of the one or more monitoring locations, the one or more monitoring locations are connected to a laser absorption spectroscopy system, or any combination thereof.
